(19) Europäisches Patentamt
European Patent Office
Office européen des brevets

(11) **EP 2 362 732 B1**

(12) **EUROPEAN PATENT SPECIFICATION**

(45) Date of publication and mention
of the grant of the patent:
**20.05.2015 Bulletin 2015/21**

(51) Int Cl.:
*A61L 2/24* (2006.01)     *A61L 2/18* (2006.01)
*A01N 63/00* (2006.01)   *A01N 63/04* (2006.01)
*A01N 59/00* (2006.01)   *A01P 1/00* (2006.01)
*C12N 7/00* (2006.01)    *C12N 9/08* (2006.01)
*C11D 3/04* (2006.01)    *C11D 3/386* (2006.01)
*C11D 3/39* (2006.01)    *C11D 3/48* (2006.01)
*C11D 11/00* (2006.01)

(21) Application number: **09779336.8**

(22) Date of filing: **22.04.2009**

(86) International application number:
**PCT/EP2009/054848**

(87) International publication number:
**WO 2010/046142 (29.04.2010 Gazette 2010/17)**

(54) **A METHOD OF OBTAINING HIGH-LEVEL DISINFECTION IN A WASHER DISINFECTOR, AND A WASHER DISINFECTOR**

VERFAHREN ZUR ERZIELUNG EINER HOCHGRADIGEN DESINFEKTION IN EINEM WASCH-DESINFEKTIONSGERÄT, UND WASCH-DESINFEKTIONSGERÄT

PROCÉDÉ D OBTENTION D UN NIVEAU ÉLEVÉ DE DÉSINFECTION DANS UN AUTOLAVEUR ET AUTOLAVEUR

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **23.10.2008 PCT/EP2008/064383**
**03.04.2009 EP 09157351**

(43) Date of publication of application:
**07.09.2011 Bulletin 2011/36**

(73) Proprietors:
• **Getinge Disinfection AB**
**351 15 Växjö (SE)**
• **Novozymes A/S**
**2880 Bagsvaerd (DK)**

(72) Inventors:
• **JOHANSSON, Jonas**
**S-352 45 Växjö (SE)**
• **CHRISTENSEN, Bjoern Eggert**
**DK-2880 Bagsvaerd (DK)**
• **ALLESEN-HOLM, Marie**
**DK-3400 Hilleroed (DK)**
• **DANIELSEN, Steffen**
**DK-2880 Bagsvaerd (DK)**

• **GJERMANSEN, Morten**
**DK-2670 Greve (DK)**

(74) Representative: **Simonsson, Erik**
**Awapatent AB**
**Västra Esplanaden 9 A**
**Box 99**
**351 04 Växjö (SE)**

(56) References cited:
**WO-A-02/08377**     **WO-A-96/38548**
**WO-A-99/08531**     **WO-A1-2007/100514**
**WO-A1-2007/147538**   **WO-A2-95/27046**
**US-A- 5 389 369**

• **HANSEN E.H. ET AL: "Curvularia haloperoxidase: Antimicrobial activity and potential application as a surface disinfectant" APPLIED AND ENVIRONMENTAL MICROBIOLOGY, vol. 69, no. 8, 1 August 2003 (2003-08-01) , pages 4611-4617, XP002512029 New York, USA**

**(Cont. next page)**

• Getinge USA Inc: "5666 Washer/Disinfector - Product Specification" The Getinge Group 26 July 2007 (2007-07-26), XP002598690 Retrieved from the Internet: URL:http://www.getinge.com/content/GETINGE -Healthcare/Documents/ProductSpecification s/Washers_PRODSPEC_8666-0707_EN_US.pdf [retrieved on 2010-08-16]

• "WASHER-DISINFECTORS - PART 1: GENERAL REQUIREMENTS, TERMS AND DEFINITIONS AND TESTS" INTERNATIONAL STANDARD - ISO, ZUERICH, CH, no. PART 01, 15 April 2006 (2006-04-15), pages 1-70, XP009067628

**Description**

**Field of the Invention**

The present invention relates to a method of disinfecting or sterilizing a device in a washer disinfector.
The present invention further relates to a washer disinfector for disinfecting or sterilizing a device.

**Technical Background**

Washer disinfectors are employed in many health-care related facilities, such as animal or human hospitals, nursing homes, dentists, research laboratories etc., for cleaning, disinfection and, optionally, drying, of, for example, solid and tubular surgical instruments, dishes, hand bowls, glass goods, suction flasks, baby bottles, anaesthesia equipment, etc., that have, or may have, come into contact with pathogenic substances. A typical example of a washer disinfector is disclosed in WO 2007/147536 or WO 2007/147538. Pathogenic substances that are particularly difficult to inactivate, or kill, in a washer disinfector include spores, viruses and bacteria.
Spores are known to form from aerobic Bacilli, anaerobic Clostridia, selected sarcinae and a few actinomycetes. Spores resemble certain plant seeds in that they do not carry out any metabolic reactions. In this regard they are especially suited to withstand severe environmental stress and are known to survive prolonged exposures to heat, drying, radiation and toxic chemicals. These properties make spores especially difficult to kill in environments, like living tissue or objects which come in contact with living tissue, which would be adversely effected by extreme conditions.
Most fungi, viruses and vegetative cells of pathogenic bacteria are sterilized within minutes at 70 degrees Celsius; many spores are sterilized at 100 degrees Celsius. However, the spores of some saprophytes can survive boiling for hours. Furthermore, some pathogenic viruses, such as polio virus, are much more difficult to inactivate than "normal" viruses. Still further, some bacteria, such as most *Mycobacteria,* are also more difficult to inactivate.
Heat is presently the most commonly used means to ensure a desired degree of disinfection of devices, including medical devices, in a washer disinfector. Typically, medical devices are treated at a temperature of 90-95°C for 1-10 minutes in the washer disinfector in order to achieve what is referred to as "high level disinfection" in the document "Class II Special Controls Guidance Document: Medical Washers and Medical Washer-Disinfectors; Guidance for the Medical Device Industry and FDA Review Staff", U.S. Food and Drug Administration, Feb. 2002, in particular section II, paragraph I "Performance Data", point 3 (d) (4) of that document.
As alternative to a heat treatment the disinfection may also be achieved by means of a chemical treatment, such as a glutaraldehyde treatment or a peracetic acid treatment.
However, many modern medical devices include complicated combinations of various sensitive materials and/or electronic appliances, such modern medical devices including, for example, endoscopes and anaesthetic equipment. Such medical devices are often sensitive to high temperatures and chemical treatment and often have a reduced service life when being repeatedly exposed to a high level disinfection step of the above described types in a washer disinfector.
Compositions comprising haloperoxidases are known to be effective for disinfecting medical devices (see WO 96/38548, WO 95/27046 and US 5,389,369). Furthermore, similar compositions that further comprise ammonium ions to provide improved disinfection in water treatments and of surfaces are known from WO 02/08377 and WO 99/08531. However, none of these documents consider their use in washer disinfector.

**Summary of the invention**

An object of the present invention is to provide a method of disinfecting or sterilizing a device in a washer disinfector, which method, in comparison with the prior art methods, is less degenerative to the device being disinfected or sterilized in the washer disinfector.
This object is achieved by means of a method of disinfecting or sterilizing a device in a washer disinfector, comprising supplying a haloperoxidase, hydrogen peroxide, chloride and/or bromide ions, and ammonium ions to the washer-disinfector.
An advantage of this invention is that a method for killing or inactivating spores and/or inactivating viruses and/or killing or inhibiting growth of bacteria, such as *Mycobacteria,* in a washer disinfector is provided, making it possible to achieve at least "high level disinfection", as defined in the above mentioned document of U.S. Food and Drug Administration, Feb. 2002, without requiring high temperatures or chemicals that might reduce the service life of the device, such as a sensitive piece of medical equipment, that is to be disinfected or sterilized.
According to one embodiment said haloperoxidase, hydrogen peroxide, chloride and/or bromide ions, and ammonium ions are supplied to a washing chamber of said washer disinfector during an enzymatic disinfection phase, said enzymatic disinfection phase comprising contacting said haloperoxidase, hydrogen peroxide, chloride and/or bromide ions, and ammonium ions with said device in said washing chamber at a temperature which is 65 degrees Celsius or lower. More preferably, at least a portion of said enzymatic disinfection phase occurs at a temperature which is in the range of 20 to 65 degrees Celsius. An advantage of this embodiment is that high level disinfection can be achieved without requiring high temperatures that may reduce the service life of the device to be disinfected, and that would also increase the energy cost of the washer disinfector.

According to one embodiment said enzymatic disinfection phase occurs for a period of time which is 5-90 minutes, said period of time starting at the moment of introducing the haloperoxidase, hydrogen peroxide, chloride and/or bromide ions, and ammonium ions into the washing chamber, and ending the moment of draining the haloperoxidase, hydrogen peroxide, chloride and/or bromide ions, and ammonium ions from the washing chamber. A period of time of 5-90 minutes has been found to be optimum for obtaining quick operation of the washer disinfector, and efficient killing or inactivation of spores and viruses.

According to one embodiment said enzymatic disinfection phase is preceded by a washing phase, during which a detergent and water is added to said washing chamber for treating said device at a temperature in the range of 20 to 85 degrees Celsius. An advantage of this embodiment is that various contaminants, such as blood, fat, tissue residues etc., are removed from said device before starting the enzymatic disinfection phase, making the latter much more efficient in killing or inactivating spores, and inactivating viruses. In case of a sensitive device being washed, the temperature in the washing chamber during the washing phase is preferably 20 to 65 degrees Celsius.

According to one embodiment said enzymatic disinfection phase is followed by a post enzymatic disinfection rinse phase, in which a cleaned water, having passed through a filter having a pore size of maximum 0.2 micrometer, or having at least the corresponding degree of purity, is supplied to said washing chamber to rinse said device. An advantage of this embodiment is that re-contamination, with for example spores, of said device after the enzymatic disinfection phase is avoided, since the cleaned rinsing water does not contain anything that might cause such contamination. In one embodiment, the cleaned water is a sterilized water.

According to one embodiment a drying phase is carried out after said enzymatic disinfection phase and before the device is removed from the washing chamber, said drying phase comprising blowing air having a temperature of 90 degrees Celsius or lower into the washing chamber. An advantage of this embodiment is that efficient drying is achieved with a limited, or no, reduction of the service life of said device. In case of a sensitive device being dried, the temperature in the washing chamber during the drying phase is preferably 20 to 65 degrees Celsius.

Preferably, the temperature inside a washing chamber of the washer disinfector is 65 degrees Celsius or lower during the entire process of sterilizing or disinfecting said device.

According to one embodiment said method comprises at least the following consecutive phases;

at least one washing phase for treating said device with a detergent,

at least one post wash rinse phase for rinsing said device with water,

at least one enzymatic disinfection phase for treating said device with said haloperoxidase, hydrogen peroxide, chloride and/or bromide ions, and ammonium ions, and

at least one post enzymatic disinfection rinse phase for rinsing said device with water.

An advantage of this method is that a very efficient disinfection is achieved, the method being effective in that sense that a short total process time can be achieved, i.e. a total process time of typically less than 120 minutes, preferably less than 60 minutes, an most preferably less than 45 minutes, from inserting the contaminated device in the washing chamber until a disinfected or sterilized device may be removed therefrom, and that a low thermal load is exerted on the device during the process. According to one embodiment, the final post enzymatic disinfection rinse phase is followed by at least one drying phase for drying said device by means of bringing it into contact with drying air.

A further object of the present invention is to provide a washer disinfector which is operative for disinfecting or sterilizing a device in a manner which is less degenerative to said device.

This object is achieved by means of a washer disinfector for disinfecting or sterilizing a device, said washer disinfector comprising

a washing chamber which is adapted for housing said device, and

an arrangement containing a haloperoxidase, hydrogen peroxide, chloride and/or bromide ions, and ammonium ions and arranged for supplying those substances to said washing chamber.

An advantage of this washer disinfector is that it is capable of achieving a high level disinfection of said device, without exposing said device to excessive thermal and/or chemical treatments.

The washer disinfector comprises a haloperoxidase supply arrangement which is operative for storing and supplying a haloperoxidase solution containing haloperoxidase, and a hydrogen peroxide supply arrangement which is operative for storing and supplying a hydrogen peroxide solution containing hydrogen peroxide and/or a hydrogen peroxide precursor. An advantage of this is that activation of the haloperoxidase occurs only when both solutions have been feed to the washing chamber, meaning that the life and activity of the haloperoxidase is maintained for an extended period of time. The chloride and/or bromide ions, and ammonium ions required for the reaction could be included in one, or both of the two solutions, or could be supplied as a third solution.

According to one embodiment the washer disinfector comprises a control unit which is operative for controlling the temperature inside said washing chamber to be 65 degrees Celsius or lower during the entire process of disinfecting or sterilizing said device. An advantage of this embodiment is that a temperature of 65 degrees Celsius, or lower, can be maintained in an easy and accurate manner, without requiring constant manual supervision.

**Brief description of the Drawings**

The invention will now be described in more detail with reference to the appended drawing in which:

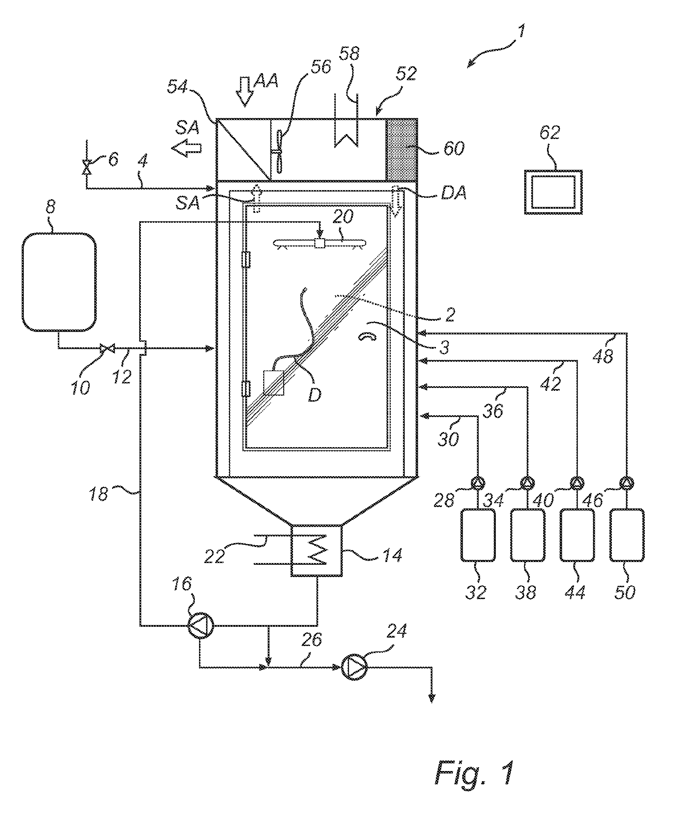

Fig. 1 is a schematic representation of a washer disinfector.

**Detailed description of preferred Embodiments**

Fig. 1 illustrates a washer disinfector 1 according to a first embodiment of the present invention. The washer disinfector 1 may be used at hospitals, surgical centers, elderly care institutions, chemical and biochemical laboratories, research institutes, pharmaceutical industries, dentists, veterinarian centers, outpatient centers etc., where devices that might have come into contact with spores and/or viruses need to be disinfected or sterilized. The washer disinfector 1 has a washing chamber 2 into which devices, such as a medical device schematically indicated as D in Fig. 1, may be introduced. A door 3, which may, for example, be a transparent glass-door, is operative for providing access to the washing chamber 2 when inserting one or several dirty devices thereto, or removing one or several cleaned devices therefrom. The devices may either be introduced as they are, or may be put on baskets supported by a wash cart, that is per se known from, for example, WO 2007/147536.

Liquid, in the form of, for example, drinking quality water, can be supplied to the washing chamber 2 by means of a tap water supply pipe 4, which is controlled by means of control valve 6. It is also possible to supply, by means of the supply pipe 4, a treated water, such as a softened water, a de-ionized water, or a water that has undergone a reverse osmosis process. Sterilized water can be supplied from a sterilized water tank 8, or from an integrated system for producing sterilized water from tap water. The sterilized water could, for example, fulfill the definition of Sterilized Water of the United States Pharmacopeia, 23d revision, 1 January 1995. As alternative, the water in the tank 8 is a cleaned water, that has passed through a filter having a pore size of maximum 0.2 micrometer, or has at least the corresponding degree of purity. Such a cleaned water is essentially free from bacteria and spores. A control valve 10 controls the supply of sterilized water to the washing chamber 2 via a pipe 12.

At the bottom of the washing chamber 2 a sump 14 is arranged. A circulation pump 16 is connected to the sump 14 by means of a pipe 18 and is operative for pumping liquid from the sump 14 to a liquid distributor 20 located inside the washing chamber 2. Hence, the circulation pump 16 is operative for circulating the liquid in the washing chamber 2. The liquid distributor 20 could, for example, comprise one or several spray arms of the type disclosed in WO 2007/147536. A heater 22 is provided in the bottom of the sump 14 for heating the circulating liquid to a desired temperature.

A drainage pump 24 is, via a pipe 26, connected to the bottom of the circulation pump 16 and to the bottom of the pipe 18. The drainage pump 24 is operative for efficiently draining liquid out of the washing chamber 2.

A detergent supply pump 28 is operative for supplying, via a pipe 30, a liquid dishwasher detergent, which may be of any suitable commercially available type, from a can 32 to the washing chamber 2.

A haloperoxidase supply pump 34 is operative for supplying, via a pipe 36, a haloperoxidase solution containing haloperoxidase, chloride and/or bromide ions, and ammonium ions from a can 38 to the washing chamber 2.

A hydrogen peroxide supply pump 40 is operative for supplying, via a pipe 42, a hydrogen peroxide solution, which may contain hydrogen peroxide and/or a hydrogen peroxide precursor, from a can 44 to the washing chamber 2.

A rinse supply pump 46 is operative for supplying, via a pipe 48, a rinse solution, which may be of any suitable commercially available type, for improving the drying of the goods inside the washing chamber, from a can 50 to the washing chamber 2.

A drying device 52 is operative for drying the devices inside the washing chamber 2 after the washing thereof. The drying device 52 is provided with a heat-exchanger 54 which is operative for heat exchanging incoming ambient air AA with outgoing spent drying air SA. The drying device 52 further comprises a fan 56, which is operative for forwarding the drying air through the drying device 52 and through the washing chamber 2, a heater 58, which is operative for heating the drying air to a suitable temperature, and a high efficiency filter 60 for filtering the air, which is then, as indicated by means of an arrow DA in Fig.1, introduced into the washing chamber 2. The high efficiency filter 60 is preferably a so called HEPA filter, which fulfills the standard ISO 15883 referring to EN 1822-1:1998, class H12 or preferably better, such as H13. The drying air, which does not contain any contaminants, is used for drying the device D, and is then exhausted as the spent drying air SA via the heat-exchanger 54.

A control unit 62 is operative for controlling the operation of the washer disinfector 1, including the operation of the valves 6 and 10, the pumps 16, 24, 28, 34, 40, 46, the heaters 22, 58 and the fan 56.

The method of obtaining high level disinfection in the washer disinfector 1 will now be described with reference to a non-limiting example, in which the various process steps are described in the order in which they would normally be performed.

Devices, such as endoscopes and other sensitive equipment represented by D, are introduced in the washing chamber 2 via the door 3, which is subsequently closed.

A pre rinse phase is then started by means of supplying water to the washing chamber 2 via the pipe 4. Hence, the control unit 62 orders the valve 6 to open. When a sufficient amount of water has been feed to the washing chamber 2 the valve 6 is closed and the pump 16 is started. The water is circulated inside the washing chamber 2 during typically 10 seconds to 10 minutes, more typically during 30 seconds to 5 minutes, to rinse off any solids from the devices D. The pre rinse phase typically takes place without any heating, which means that the water being circulated inside the washing chamber 2 has a temperature of typically 5 - 20 degrees Celsius. It is also possible, as an option, to heat the water, by means of the heater 22, to a temperature of typically 20-50 degrees Celsius. After the pre rinse phase, the pump 16 is stopped, and the drainage pump 24 is started to drain liquid out of the washing chamber 2.

A washing phase is then started by supplying water to the washing chamber 2, via the pipe 4. The pump 16 is then started, and the heater 22 is activated to heat the circulating water. When the circulating water has been heated to typically 15-35 degrees Celsius the detergent supply pump 28 is started to supply dishwasher detergent to the washing chamber 2. The detergent could be of any suitable type being effective for removing, for example, fat, blood and tissue residues from the device D. An example of a suitable detergent is "Getinge Power Wash", which is available from Getinge Aktiebolag, Getinge, Sweden. The heater 22 then heats the circulating mixture of water and detergent further to the washing temperature, which is preferably a temperature in the range of 20-85 degrees Celsius. In case of a sensitive device being washed, the temperature in the washing chamber during the washing phase is preferably 20 to 65 degrees Celsius. The mixture of water and detergent is then circulated, at said washing temperature in the range of 20-85 degrees Celsius, or 20 to 65 degrees Celsius as the case may be, in the washing chamber 2 during typically 30 seconds to 15 minutes, more preferably during 1-5 minutes, by means of the pump 16. After the washing phase, the pump 16 is stopped, and the drainage pump 24 is started to drain liquid out of the washing chamber 2.

A post wash rinse phase is then started by opening the valve 4 to supply water to the washing chamber 2. The water is then circulated, by means of the pump 16, during, typically, 10 seconds to 5 minutes in the washing chamber 2, and is then drained by means of the drainage pump 24. Optionally the post wash rinse phase is repeated once, twice, or more times. Optionally, the heater 22 could be activated for heating the water during the post wash rinse phase to a temperature of 20-50 degrees Celsius.

An enzymatic disinfection phase is then started by supplying water to the washing chamber 2, via the pipe 4. The pump 16 is then started, and the heater 22 is activated to heat the circulating water. When the circulating water has been heated to typically 15-35 degrees Celsius the haloperoxidase supply pump 34 and the hydrogen peroxide supply pump 40 are started to supply haloperoxidase solution and hydrogen peroxide solution, respectively, to the washing chamber 2. The hydrogen peroxide activates the haloperoxidase, and by keeping those substances separated from each other, in separate solutions, until they are to co-operate in the enzymatic disinfection phase, prolongs the life of both solutions. The heater 22 then heats the circulating mixture of water, haloperoxidase solution and hydrogen peroxide solution further to the enzymatic disinfection phase temperature, which is a temperature in the range of 20-65 degrees Celsius, preferably 20-60 degrees Celsius, and most preferably 20-55 degrees Celsius. The mixture of water, haloperoxidase solution and hydrogen peroxide solution is then circulated in the washing chamber 2 by means of the pump 16 during typically 1-120 minutes, preferably during 10-90 minutes, more preferably during 10-60 minutes, and even more preferably during 20-60 minutes. After the enzymatic disinfection phase, the pump 16 is stopped, and the drainage pump 24 is started to drain liquid out of the washing chamber 2.

A post enzymatic disinfection rinse phase is then started, preferably by opening the valve 10 to supply sterilized water from the tank 8 to the washing chamber 2. The sterilized water is then circulated, by means of the pump 16, during, typically, 10 seconds to 10 minutes, preferably during 30 seconds to 5 minutes, in the washing chamber 2, and is then drained by means of the drainage pump 24. Optionally, the rinse supply pump 46 may be started to supply rinse solution to the washing chamber 2. Optionally, the post enzymatic disinfection rinse phase is repeated once, twice, or more times. Optionally, the heater 22 could be activated for heating the sterilized water during the post enzymatic disinfection rinse phase to a temperature of 20-50 degrees Celsius.

Optionally, a thermal disinfection phase could be employed to further enhance the degree of disinfection in case of disinfecting devices that are less sensitive to high temperatures. If a thermal disinfection phase is employed, water is supplied, either as tap water via the pipe 4 or as sterilized water via the pipe 12, to the washing chamber 2. The water is then circulated by means of the pump 16 and is heated to a temperature of typically 75-95 degrees Celsius, more typically 85-92 degrees Celsius, by means of the heater 22. The circulating water is then kept at this temperature for a stipulated period of time, for example 1 minute at 90 degrees Celsius, to obtain a further disinfection. Optionally, the rinse supply pump 46 may be started to supply rinse solution to the washing chamber 2. After the thermal disinfection phase, the pump 16 is stopped, and the drainage pump 24 is started to drain liquid out of the washing chamber 2. If a thermal disinfection phase is included, the post enzymatic disinfection rinse phase would normally be performed with tap water instead of sterilized water.

Optionally, a drying phase is then started by means of starting the fan 56 and the heater 58. The drying air DA is, typically, heated to a temperature of 50-120 degrees Celsius. If sensitive devices, such as endoscopes, are present inside the washing chamber 2 the temperature of the drying air is preferably kept at about 90 degrees Celsius or lower, more preferably at 65 degrees Celsius or lower, and most preferably below about 60 degrees Celsius. Typically, the drying phase lasts for about 3-60 minutes, more preferably for 5-45 minutes, and most preferably for 10-30 minutes.

After the drying phase the door 3 is opened, and the disinfected devices D are removed from the washing chamber 2.

It will be appreciated that many variants of the above described embodiments are possible within the scope of the appended claims.

Hereinabove it has been described, for example, that the dish washer detergent and the haloperoxidase and hydrogen peroxide solutions, respectively, are supplied to the washing chamber 2 already before the desired temperature of the respective phase has been reached. While this is a preferred embodiment, which saves operating time, it is also possible to wait with supplying the respective solution until the desired operating temperature of the respective phase has been reached. It is also possible, but often less preferred, to supply the respective solution at the same time as, or even before, supplying the water needed for the respective phase to the washing chamber 2.

Hereinabove, the water supplied via the pipe 4 is referred to as "tap water" by what is meant a water of such a quality that local authorities would accept it for drinking by humans on a daily basis, for example a water that fulfills "Guidelines for Drinking Water Quality, 3rd Edition" published by WHO. It will be appreciated that other water types, less pure than drinking water, may also be utilized, in particular in the earlier phases, such as in the pre rinse phase and the washing phase. On the other hand, the water used, in particular in the washing phase, could preferably be softened or de-ionized, to improve the effect of the detergent.

Hereinabove it has been described that sterilized water is supplied from a tank 8. It will be appreciated that a water cleaning device, which cleans tap water to a sterilized water quality by means of filtering it, could also be integrated with the washer disinfector 1, or could be arranged separately next to the washer disinfector 1. Furthermore, it is also possible, as alternative, to utilize a filter having a pore size of maximum 0.2 micrometer and being included in the washer disinfector, or being arranged next to the washer disinfector, to clean tap water before using it in the post enzymatic disinfection rinse phase. Such a cleaned water would, just as a sterilized water, be substantially free of spores.

Hereinabove, it has been described that two solutions, i.e., a haloperoxidase solution containing haloperoxidase, chloride and/or bromide ions, and ammonium ions, and a hydrogen peroxide solution containing hydrogen peroxide and/or a hydrogen peroxide precursor are utilized. As an alternative, it is also possible to have one, or more, of the chloride and/or bromide ions, and ammonium ions, in the hydrogen peroxide solution, either in combination with having those ions in the haloperoxidase solution, or instead of having them in the haloperoxidase solution. It would also be possible to utilize more than two solutions, such as three solutions. In the latter case a first solution could comprise haloperoxidase, a second solution could comprise hydrogen peroxide and/or a hydrogen peroxide precursor, and a third solution could comprise chloride and/or bromide ions, and ammonium ions. It is also possible to have even further solutions, but this is normally less preferred due to the more costly design of the washer disinfector with more dosage devices. Further components, such as buffers and surfactants, may also be added to any of the above mentioned solutions.

A further option is to formulate the haloperoxidase, the hydrogen peroxide, and/or the hydrogen peroxide precursor, the chloride and/or bromide ions, and the ammonium ions in the form of a tablet, which is manually or automatically inserted into the washing chamber at the start of the enzymatic disinfection phase. For example, tablets could be fed from a magazine which is controlled by the control unit 62 to supply a tablet to the washing chamber at the start of the enzymatic disinfection phase. A still further option is to formulate the haloperoxidase, the hydrogen peroxide and/or the hydrogen peroxide precursor, the chloride and/or bromide ions, and the ammonium ions in the form of a powder, which is manually or automatically inserted into the washing chamber, for example from a specific compartment inside the washer disinfector, at the start of the enzymatic disinfection phase.

It will be appreciated that the equipment for dosing the haloperoxidase, the hydrogen peroxide hydrogen peroxide, chloride and/or bromide ions, and ammonium ions could both be provided in a new washer disinfector, but could also be utilized for retrofitting an existing washer disinfector.

## Detailed description of the Chemical reactions in the Enzymatic disinfection phase

Haloperoxidases and Compounds Exhibiting Haloperoxidase Activity

The haloperoxidases suitable for being incorporated in the method of the invention include chloroperoxidases, bromoperoxidases and compounds exhibiting chloroperoxidase or bromoperoxidase activity. Haloperoxidases form a class of enzymes, which are capable of oxidizing halides (Cl-, Br-, I-) in the presence of hydrogen peroxide or a hydrogen peroxide generating system to the corresponding hypohalous acids.

Haloperoxidases are classified according to their specificity for halide ions. Chloroperoxidases (E.C. 1.11.1.10) catalyze formation of hypochlorite from chloride ions, hypobromite from bromide ions and hypoiodite from iodide ions; and bromoperoxidases catalyze formation of hypobromite from bromide ions and hypoiodite from iodide ions. Hypoiodite, however, undergoes spontaneous disproportionation to iodine and thus iodine is the observed product. These hypohalite compounds may subsequently react with other compounds forming halogenated compounds.

In a preferred embodiment, the haloperoxidase of the invention is a chloroperoxidase.

Haloperoxidases have been isolated from various organisms: mammals, marine animals, plants, algae, lichen, fungi and bacteria. It is generally accepted that haloperoxidases are the enzymes responsible for the formation of halogenated compounds in nature, although other enzymes may be involved.

Haloperoxidases have been isolated from many different fungi, in particular from the fungus group dematiaceous hyphomycetes, such as Caldariomyces, e.g., C. fumago, Alternaria, Curvularia, e.g., C. verruculosa and C. inaequalis, Drechslera, Ulocladium and Botrytis.

Haloperoxidases have also been isolated from bacteria such as Pseudomonas, e.g., P. pyrrocinia and Streptomyces, e.g., S. aureofaciens.

In a preferred embodiment, the haloperoxidase is a vanadium haloperoxidase (i.e. a vanadium or vanadate containing haloperoxidase) derivable from Curvularia sp., in particular Curvularia verruculosa or Curvularia inaequalis, such as C. inaequalis CBS 102.42 as described in WO 95/27046, e.g. a vanadium haloperoxidase encoded by the DNA sequence of WO 95/27046, figure 2 all incorporated by reference; or C. verruculosa CBS 147.63 or C. verruculosa CBS 444.70 as described in WO 97/04102. Preferably, the amino acid sequence of the haloperoxidase has at least 90% identity, preferably 95% identity to the amino acid sequence of a haloperoxidase obtainable from Curvularia verruculosa (see e.g. SEQ ID NO:2 in WO 97/04102) or Curvularia inequalis (e.g. the mature amino acid sequence encoded by the DNA sequence in figure 2 of WO 95/27046).

In another preferred embodiment the haloperoxidase is a vanadium containing haloperoxidase; in particular a vanadium chloroperoxidase. The vanadium chloroperoxidase may be derivable from Drechslera hartlebii as described in WO 01/79459, Dendryphiella salina as described in WO 01/79458, Phaeotrichoconis crotalarie as described in WO 01/79461, or Geniculosporium sp. as described in WO 01/79460. The vanadium haloperoxidase is more preferably derivable from Drechslera hartlebii (DSM 13444), Dendryphiella salina (DSM 13443), Phaeotrichoconis crotalarie (DSM 13441) or Geniculosporium sp. (DSM 13442).

The concentration of the haloperoxidase is typically in the range of 0.01-100 ppm enzyme protein, preferably 0.05-50 ppm enzyme protein, more preferably 1-40 ppm enzyme protein, more preferably 0.1-20 ppm enzyme protein, and most preferably 0.5-10 ppm enzyme protein.

In an embodiment, the concentration of the haloperoxidase is typically in the range of 5-50 ppm enzyme protein, preferably 5-40 ppm enzyme protein, more preferably 8-32 ppm enzyme protein.

Determination of Haloperoxidase Activity

An assay for determining haloperoxidase activity may be carried out by mixing 100 $\mu$L of haloperoxidase sample (about 0.2 $\mu$g/mL) and 100 $\mu$L of 0.3 M sodium phosphate pH 7 buffer - 0.5 M potassium bromide - 0.008% phenol red, adding the solution to 10 $\mu$L of 0.3% $H_2O_2$ and measuring the absorption at 595 nm as a function of time.

Another assay using monochlorodimedone (Sigma M4632, $\varepsilon$ = 20000 M-1 cm-1 at 290 nm) as a substrate may be carried out by measuring the decrease in absorption at 290 nm as a function of time. The assay is done in an aqueous solution of 0.1 M sodium phosphate or 0.1 M sodium acetate, 50 $\mu$M monochlorodimedone, 10 mM KBr/KCl, 1 mM $H_2O_2$ and about 1 $\mu$g/mL haloperoxidase. One haloperoxidase unit (HU) is defined as 1 micromol of monochlorodimedone chlorinated or brominated per minute at pH 5 and 30°C.

Hydrogen Peroxide

The hydrogen peroxide required by the haloperoxidase may be provided as an aqueous solution of hydrogen peroxide or a hydrogen peroxide precursor for in situ production of hydrogen peroxide. Any solid entity which liberates upon dissolution a peroxide which is useable by haloperoxidase can serve as a source of hydrogen peroxide. Compounds which yield hydrogen peroxide upon dissolution in water or an appropriate aqueous based medium include but are not limited to metal peroxides, percarbonates, persulphates, perphosphates, peroxyacids, alkyperoxides, acylperoxides, peroxyesters, urea peroxide, perborates and peroxycarboxylic acids or salts thereof.

Another source of hydrogen peroxide is a hydrogen peroxide generating enzyme system, such as an oxidase together with a substrate for the oxidase. Examples of combinations of oxidase and substrate comprise, but are not limited to, amino acid oxidase (see e.g. US 6,248,575) and a suitable amino acid, glucose oxidase (see e.g. WO 95/29996) and glucose, lactate oxidase and lactate, galactose oxidase (see e.g. WO 00/50606) and galactose, and aldose oxidase (see e.g. WO 99/31990) and a suitable aldose.

By studying EC 1.1.3._, EC 1.2.3._, EC 1.4.3._, and EC 1.5.3._ or similar classes (under the International Union of Biochemistry), other examples of such combinations of oxidases and substrates are easily recognized by one skilled in the art.

Hydrogen peroxide or a source of hydrogen peroxide may be added at the beginning of or during the process, e.g., typically in an amount corresponding to levels of from 0.001 mM to 25 mM, preferably to levels of from 0.005 mM to 5 mM, and particularly to levels of from 0.01 to 1 mM hydrogen peroxide. Hydrogen peroxide may also be used in an amount corresponding to levels of from 0.1 mM to 25 mM, preferably to levels of from 0.5 mM to 15 mM, more preferably to levels of from 1 mM to 10 mM, and most preferably to levels of from 2 mM to 8 mM hydrogen peroxide.

Chloride and/or Bromide ions

According to the invention, the chloride and/or bromide ions (Cl- and/or Br-) needed for the reaction with the haloperoxidase may be provided in many different ways, such as by adding salts of chloride and/or bromide. In a preferred embodiment the salts of chloride and bromide are sodium chloride (NaCl), sodium bromide (NaBr), potassium chloride (KCl), potassium bromide (KBr), ammonium chloride (NH4Cl) or ammonium bromide (NH4Br); or mixtures thereof.

In an embodiment, the chloride and/or bromide ions are limited to only chloride ions (Cl-) or bromide ions (Br-). In another embodiment, the chloride and/or bromide ions are limited to only chloride ions (Cl-) and bromide ions (Br-). The chloride ions may be provided by adding a salt of chloride to an aqueous solution. The salt of chloride may be sodium chloride, potassium chloride or ammonium chloride; or a mixture thereof.

The bromide ions may be provided by adding a salt of bromide to an aqueous solution. The salt of bromide may be sodium bromide, potassium bromide or ammonium bromide; or a mixture thereof.

The concentration of each of chloride and bromide ions are typically in the range of from 0.01 mM to 1000 mM, preferably in the range of from 0.05 mM to 500 mM, more preferably in the range of from 0.1 mM to 100 mM, most preferably in the range of from 0.1 mM to 50 mM, and in particular in the range of from 1 mM to 25 mM. The concentration of chloride ions is independent of the concentration of bromide ions; and vice versa.

In an embodiment, the molar concentration of each of chloride and bromide ions is at least two times higher, preferably at least four times higher, more preferably at least six times higher, most preferably at least eight times higher, and in particular at least ten times higher than the concentration of ammonium ions.

Ammonium ions

The ammonium ions (NH4+) needed to kill or inactivate microbial spores and/or to inactivate viruses according to the methods of the invention may be provided in many different ways, such as by adding a salt of ammonium. In a preferred embodiment the ammonium salt is ammonium sulphate ((NH4)2SO4), ammonium carbonate ((NH4)2CO3), ammonium chloride (NH4Cl), ammonium bromide (NH4Br), or ammonium iodide (NH4I); or a mixture thereof.

The concentration of ammonium ions is typically in the range of from 0.01 mM to 1000 mM, preferably in the range of from 0.05 mM to 500 mM, more preferably in the range of from 0.1 mM to 100 mM, most preferably in the range of from 0.1 mM to 50 mM, and in particular in the range of from 1 mM to 25 mM.

Microbial Spores

The microbial spores which are killed or inactivated with a haloperoxidase, hydrogen peroxide, chloride or bromide ions, and ammonium ions according to the invention comprise all kinds of spores.

In an embodiment the microbial spores are endospores, such as all Clostridium sp. spores, Brevibacillus sp. spores and Bacillus sp. spores, e.g. spores from Bacillus anthracis, Bacillus cereus, Bacillus subtilis, Bacillus putida, and Bacillus pumila.

In another embodiment the microbial spores are exospores, such as Actinomycetales spores, e.g. spores from Actinomyces sp., Streptomyces sp., Thermoactinomyces sp., Saccharomonospora sp., and Saccharopylospora sp.

In another embodiment the microbial spores are bacterial spores. Examples of bacterial spores include, but are not limited to, all Clostridium sp. spores and Bacillus sp. spores as mentioned above.

In yet another embodiment the microbial spores are fungal spores. Examples of fungal spores include, but are not limited to, conidiospores, such as spores from Aspergillus sp., and Penicillium sp.

Viruses

The viruses, which are inactivated with a haloperoxidase, hydrogen peroxide, chloride ions and/or bromide ions, and ammonium ions according to one embodiment of the invention, comprise all kinds of viruses.

In an embodiment the viruses are selected from the group consisting of:

Adenoviruses, Arenaviruses, Bunyaviruses, Caliciviruses, Coronaviruses, Deltaviruses, Filoviruses, Flaviviruses, Hepadnaviruses, Herpesviruses, Orthomyxoviruses, Papovaviruses, Paramyxoviruses, Parvoviruses, Picornaviruses, Poxiviruses, Rhabdoviruses, Reoviruses, Retroviruses, and Togaviruses.

In another embodiment the virus is selected from the group consisting of:

Norovirus (Norwalk virus), Poliovirus, Rotavirus, Respiratory Syncytial Virus, Rhinovirus, Parainfluenza Virus, Coronavirus, Influenza A and B viruses, Human Immunodeficiency Virus (HIV), Hepatitis A virus, Hepatitis B virus, Hepatitis C virus, Herpes simplex virus type 1, and Herpes simplex virus type 2.

In another embodiment the viruses are small non-enveloped viruses. Examples of small non-enveloped viruses include, but are not limited to, picornaviruses, such as human rhinovirus A, human rhinovirus B, Foot-and-mouth disease virus, Hepatitis A virus, and enteroviruses (such as poliovirus).

*Mycobacteria*

The Mycobacteria which are killed or inactivated with a haloperoxidase, hydrogen peroxide, chloride ions and/or bromide ions, and ammonium ions according to the invention, may be any *Mycobacterium* sp., such as species from the *Mycobacterium tuberculosis complex* (MTBC).

In an embodiment, the *Mycobacteria* of the invention are capable of causing tuberculosis.

In another embodiment, the *Mycobacteria* are selected from the group consisting of *M. tuberculosis, M. bovis, M. bovis* BCG, *M. africanum, M. microti, M. canetti, M. caprae* and *M. pinnipedii.*

In a prefered embodiment, the *Mycobacteria* according to the invention are *Mycobacterium tuberculosis* or *Mycobacterium bovis cells.*

Surfactants

The method of the invention may include application of a surfactant (for example, as part of a detergent formulation or as a wetting agent). Surfactants suitable for being applied may be non-ionic (including semi-polar), anionic, cationic and/or zwitterionic; preferably the surfactant is anionic (such as linear alkylbenzenesulfonate, alpha-olefinsulfonate, alkyl sulfate (fatty alcohol sulfate), alcohol ethoxysulfate, secondary alkanesulfonate, alpha-sulfo fatty acid methyl ester, alkyl- or alkenylsuccinic acid or soap) or non-ionic (such as alcohol ethoxylate, nonylphenol ethoxylate, alkylpolyglycoside, alkyldimethylamineoxide, ethoxylated fatty acid monoethanolamide, fatty acid monoethanolamide, polyhydroxy alkyl fatty acid amide, or N-acyl N-alkyl derivatives of glucosamine ("glucamides")), or a mixture thereof.

When included in the method of the invention, the concentration of the surfactant will usually be from about 0.01% to about 10%, preferably about 0.05% to about 5%, and more preferably about 0.1 % to about 1 % by weight. Typically, the surfactants would be included in the above mentioned haloperoxidase solution. However, the surfactants could also be included in the hydrogen peroxide solution, and/or could be included in a separate solution, or in a tablet.

Methods and Uses

In a first aspect, the present invention provides an enzymatic method for killing or inactivating spores and/or a virus and/or bacteria, such as *Mycobacteria,* comprising contacting the spores and/or virus and/or bacteria, such as *Mycobacteria,* with a composition which includes a haloperoxidase, a source of hydrogen peroxide, chloride ions and/or bromide ions, and ammonium ions in a washer disinfector. In a preferred embodiment the present invention provides a method for disinfecting or sterilizing medical devices or equipment in a washer disinfector, which method comprises contacting the medical devices or equipment with the composition in a washing chamber of a washer disinfector.

The composition may be formulated as a liquid (e.g. aqueous) or a dry product formulation. The dry product formulation may subsequently be rehydrated to form an active liquid or semi-liquid formulation usable in the method of the invention.

When the composition is formulated as a dry formulation, the components may be mixed, arranged in discrete layers or packed separately.

In the context of the present invention the term "killing or inactivating spores" is intended to mean that at least 99% of the spores are not capable of transforming (germinating) into vegetative cells. Preferably 99.9%, more preferably 99.99%, most preferably 99.999%, and in particular 99.9999% of the spores are not capable of transforming into vegetative cells. In the context of the present invention the term "inactivating viruses" is intended to mean that at least 99% of the viruses are not capable of infecting suitable cells. Preferably 99.9%, more preferably 99.99%, most preferably 99.999%, and in particular 99.9999% of the viruses are not capable of infecting suitable cells. In the context of the present invention, the term "killing or inhibiting growth of bacteria, such as *Mycobacteria,* " is intended to mean that at least 99% of the bacteria, such as *Mycobacteria,* are not viable after the treatment. Preferably 99.9%, more preferably 99.99%, most preferably 99.999%, and in particular 99.9999% of the bacteria, such as *Mycobacteria,* are not viable.

The term "disinfecting" or "disinfection" refers to "high level disinfection" according to "Content and Format of Premarket Notification [510(k)] Submissions for Liquid Chemical Sterilants/High Level Disinfectants", U.S. Food and Drug Administration, Jan. 2000, to which document the above mentioned "Class II Special Controls Guidance Document: Medical Washers and Medical Washer-Disinfectors; Guidance for the Medical Device Industry and FDA Review Staff', U.S. Food and Drug Administration, Feb. 2002, section II, paragraph I "Performance Data", point 3 (d) refers.

The methods according to the invention may be carried out at a temperature between 0 and 90 degrees Celsius, preferably between 0 and 80 degrees Celsius, more preferably between 0 and 70 degrees Celsius, still more preferably between 10 and 65 degree Celsius, even more preferably between 15 and 65 degrees Celsius, even more preferably between 20 and 65 degrees Celsius, most preferably between 20 and 60 degrees Celsius, and in particular between 20 and 55 degrees Celsius.

The methods i.e., the contacting of a device with haloperoxidase, hydrogen peroxide, chloride and/or bromide ions, and ammonium ions to disinfect or sterilize the device, may employ a treatment time of from 1 minute to at least 4 hours, preferably 10 minutes to (at least) 4 hours, more preferably from 15 minutes to (at least) 3 hours, even more preferably from 20 minutes to (at least) 2 hours, most preferably from 20 minutes to (at least) 1 hour, and in particular from 30 minutes to (at least) 1 hour.

The method is suitable for killing or inactivating spores and/or inactivating viruses and/or killing or inhibiting growth of bacteria, such as *Mycobacteria,* in a variety of environments. The method of the invention may desirably be used in any environment to reduce spore contamination and/or virus infections, such as the health-care industry (e.g. animal hospitals, human hospitals, animal clinics, human clinics, nursing homes, day-care facilities for children or senior citizens, dentists etc.), the food industry (e.g. restaurants, food-processing plants, food-storage plants, grocery stores, etc.), the hospitality industry (e.g. hotels, motels, resorts, cruise ships, etc.), the education industry (e.g. schools and universities), etc.

Due to the relatively low temperatures being utilized by the methods of the invention, they are very useful for disinfecting or sterilizing equipment, such as medical devices (e.g. dry surgical instruments, anesthesia equipment, hollowware etc), used in the health-care industry. The disinfected or sterilized equipment will exhibit reduced deformations and wear, and the equipment is ready for use substantially immediately after disinfection or sterilization. This is especially advantageous when disinfecting or sterilizing complex or heat sensitive medical devices such as ultrasound transducers and endoscopes comprising different materials, because the wear of these devices have been reduced significantly, which results in longer service life of these often very costly devices, which effectively reduces their operational cost. Indeed, even other non-medical types of equipment such as reusable hygienic articles may be disinfected or sterilized effectively by use of the present invention.

In a preferred embodiment, the disinfection or sterilization of medical devices and/or non-medical types of equipment takes place in a (Medical) Washer-Disinfector according to EN ISO 15883-1 (or as described in "Class II Special Controls Guidance Document: Medical Washers and Medical Washer-Disinfectors; Guidance for the Medical Device Industry and FDA Review Staff', U.S. Food and Drug Administration, Feb. 2002), using the methods of the invention. A detailed description of the washer-disinfector in accordance with one embodiment of the invention has been included hereinabove.

The present invention is further described by the following examples which should not be construed as limiting the scope of the invention.

## Examples

Chemicals used as buffers and substrates were commercial products of at least reagent grade. In the following Examples, the symbol "-" means "not determined".

## EXAMPLE 1

### Spore production

Streak a Tryptose Blod Agar Base (TBAB) plate from a fresh culture of *Bacillus globigii* or *B. thuringiensis* (*Bacillus thuringiensis* type strain ATCC10792). Incubate the culture overnight at 30 degrees Celsius.

Suspend a loopfull of pure *Bacillus* from the TBAB plate and suspend the cells in 2 mL of sterile water. Inoculate 2xSG plates with 100 µL of the cell suspension on each. The composition of 2xSG is as follows: 16 g/L Difco Bacto Nutrient Broth, 0.5 g/L $MgSO_4$ x $7H_2O$, 2.0 g/L KCl, 1.0 mL/100 mL of 10% glucose, 0.1 mL/100 mL of 1 M $Ca(NO_3)_2$, 0.1 mL/100 mL of 0.1 M $MnSO_4$, 10 µL/100 mL of 0.01 M $FeSO_4$, and 1% Difco Bacto Agar. Incubate plates for 48-72 hours at 30 degrees Celsius. Check for sporulation with phase-contrast microscopy. Spores are phase-bright.

When sporulation efficiency is close to 100%, harvest the cell lawn with water and suspend the cells by intensive vortexing. Collect cells by centrifugation for 5-10 minutes at 6000 G at 4 degrees Celsius. Wash cells 3 times with ice cold water. The pellet contains vegetative cells and spores.

Apply a step-density gradient for separation of the spores from the vegetative cells. Prepare for each washed pellet a centrifuge tube containing 30 mL 43% Urographin®. Prepare 3 mL of cell spore mixture in Urographin so that the final Urographin concentration is 20%. Gently load the 20% Urographin cell/spore mixture onto the top layer of the 43% Urographin.

Centrifuge at 10000 G at room temperature for 30 minutes. Gently remove supernatant.

Suspend the pure spore pellet in 1 mL ice-cold water and transfer to a microfuge tube. Centrifuge at maximum speed for 1-2 min at 4 degrees Celsius, wash pellet in ice-cold water 2 more times.

Check purity and number of spores/ml by phase contrast microscopy and a haemocytometer. Store spores suspended in water at -20 degrees Celsius.

## EXAMPLE 2

Killing of *Bacillus* spores in liquid preparations

The following reagents were prepared:

- DMG buffer (DiMethylGlutamic acid, Sigma D4379), 100 mM, pH adjusted to 6.0 with NaOH;
- $1x10^8$ *Bacillus atrophaeus* spores per mL (*Bacillus atrophaeus* spores: SUS-1-8 Log. Raven Labs, www.Ravenlabs.com), suspended in MilliQ water;
- $1x10^8$ *Bacillus thuringiensis* spores per mL (made according to Example 1), suspended in MilliQ water;
- 50 mg/L Haloperoxidase from *Curvularia verrucolosa* (see WO 97/04102) in MilliQ water;
- 800 mM Sodium chloride (NaCl) in MilliQ water;
- 100 mM Ammonium sulphate ($(NH_4)_2SO_4$) in MilliQ water;
- 10 mM Hydrogen peroxide ($H_2O_2$) in MilliQ water; and
- LB plates were made from 37 g LB Agar (Merck 0283) dissolved in 1000 mL water.

In a vial the following was mixed:

10 µL spore suspension,
250 µL DMG buffer,
40 µL NaCl solution,
40 µL $(NH_4)_2SO_4$ solution, and
20 µL Haloperoxidase solution.

The reaction was started with addition of 40 µL Hydrogen peroxide.
A vial with 10 µL spore suspension and 490 µL MilliQ water acted as a control.

The vials were incubated at room temperature (approximately 23°C) for 110 minutes. After that a dilution series was made in MilliQ water, and 100 µL from the dilutions $10^0$ to $10^{-5}$ were plated (in duplicate) onto LB agar plates. The plates were incubated for 48 hours at 33°C, and the average number of colony forming units (CFU) on each pair of duplicate plates was registered (shown in Table 1).

Table 1.

| Dilution | *Bacillus atrophaeus* spores | | *Bacillus thuringiensis* spores | |
|---|---|---|---|---|
| | Control | Treated | Control | treated |
| $10^0$ | >>200 | 0 | >>200 | 0 |
| $10^{-1}$ | >>200 | 0 | >>200 | 0 |
| $10^{-2}$ | >>200 | 0 | >>200 | 0 |
| $10^{-3}$ | > 200 | 0 | 99 | 0 |

(continued)

| Dilution | *Bacillus atrophaeus* spores | | *Bacillus thuringiensis* spores | |
|---|---|---|---|---|
| | Control | Treated | Control | treated |
| $10^{-4}$ | 70 | 0 | 11 | 0 |
| $10^{-5}$ | 4 | 0 | 1 | 0 |

The results shown in Table 1 indicate that the haloperoxidase solution of the invention has a clear and significant sporicidal effect. The number of spores able to germinate after the treatment was reduced at least 5 log units.

## EXAMPLE 3

Killing of *Bacillus* spores on a stainless steel surface

The following reagents were prepared:

- DMG buffer (DiMethylGlutamic acid, Sigma D4379), 50 mM, pH adjusted to 7.0 with NaOH;
- 200 mg/L Haloperoxidase from *Curvularia verrucolosa* (see WO 97/04102) in 50 mM DMG buffer;
- 400 mM Sodium chloride (NaCl) in MilliQ water;
- 100 mM Ammonium sulphate ($(NH_4)_2SO_4$) in MilliQ water;
- 10 mM Hydrogen peroxide ($H_2O_2$) in MilliQ water;
- 5% Sodium thiosulphate ($Na_2S_2O_3$)
- tubes containing 5 mL of 1% Tween 80 in water and approximately 1 mL glass beads (3 mm); and
- LB plates were made from 37 g LB Agar (Merck 0283) dissolved in 1000 mL water.

Stainless steel discs each containing $10^6$ *Bacillus atrophaeus* spores (Raven Labs cat. # 1-6100ST) were used for the experiment (one disc was used for one treatment).

In a vial the following components were mixed:

373.75 $\mu$L DMG buffer,
31.25 $\mu$L NaCl solution,
25 $\mu$L $(NH_4)_2SO_4$ solution, and
20 $\mu$L Haloperoxidase solution

One *Bacillus atrophaeus* spore disc ($10^6$ spores).

The reaction was started with addition of 50 $\mu$L Hydrogen peroxide. A vial with a spore disc and 500 $\mu$L DMG buffer acted as a control.

The vials were incubated at 40°C for 45 minutes. To stop the reaction 500 $\mu$L Sodium thiosulphate was added, and incubated for 15 minutes at room temperature (approximately 23°C). Each disc was then transferred to a tube containing Tween 80 and glass beads, and the tubes were shaken for 15 minutes at 300 rpm.

After that a dilution series was made in MilliQ water, and 500 $\mu$L from the dilutions $10^{-1}$ to $10^{-3}$ were plated onto LB agar plates (14 cm plates). The remaining liquid from the $10^0$ solution (approximately 4.9 mL) was plates out on big LB plates (square, 20 x 20 cm plates). The plates were incubated for 48 hours at 37°C, and the average number of colony forming units (CFU) on each plate was registered (shown in Table 2).

Table 2.

| Dilution | *Bacillus atrophaeus* spores (CFU on plates) | |
|---|---|---|
| | Control | Treated |
| $10^0$ (4,9 mL) | >>1000 | 0 |
| $10^{-1}$ (500 $\mu$L) | >>200 | 0 |
| $10^{-2}$ (500 $\mu$L) | >>200 | 0 |
| $10^{-3}$ (500 $\mu$L) | 100 | 0 |
| Calculated number of spores left on disc after treatment | 1,000,000 spores/disc | 0 spores/disc |

The results shown in Table 2 indicate that the haloperoxidase solution of the invention has a clear and significant sporicidal effect. The number of spores able to germinate after the treatment was reduced 6 log units.

## EXAMPLE 4

<u>Killing of *Bacillus* spores on a surface including use of a commercial cleaning agent</u>
The following reagents were prepared:

- Commercial cleaning agent (pH 7.0) diluted to the specified working solution with water;
- DMG buffer (DiMethylGlutamic acid, Sigma D4379), 50 mM, pH adjusted to 7.0 with NaOH;
- 200 mg/L Haloperoxidase from *Curvularia verrucolosa* (see WO 97/04102) in 50 mM DMG buffer;
- 400 mM Sodium chloride (NaCl) in MilliQ water;
- 100 mM Ammonium sulphate ($(NH_4)_2SO_4$) in MilliQ water;
- 10 mM Hydrogen peroxide ($H_2O_2$) in MilliQ water;
- 5% Sodium thiosulphate ($Na_2S_2O_3$)
- tubes containing 5 mL of 1% Tween 80 in water and approximately 1 mL glass beads (3mm); and
- LB plates were made from 37 g LB Agar (Merck 0283) dissolved in 1000 mL water.

Stainless steel discs each containing $10^6$ *Bacillus atrophaeus* spores (Raven Labs cat. # 1-6100ST) were used for the experiment (one disc was used for one treatment).
Three vials were prepared as indicated in Table 3. The reaction in Vial 1 and Vial 2 was started with addition of 50 $\mu$L Hydrogen peroxide.

Table 3.

| Vial 1 | Vial 2 | Vial 3 (control) |
|---|---|---|
| - 350 $\mu$L DMG buffer, | - 350 $\mu$L Cleaning agent, | - 500 $\mu$L Cleaning agent, |
| - 62.5 $\mu$L NaCl solution, | - 62.5 $\mu$L NaCl solution, | - a *Bacillus atrophaeus* spore disc ($10^6$ spores). |
| - 25 $\mu$L $(NH_4)_2SO_4$ solution | - 25 $\mu$L $(NH_4)_2SO_4$ solution | |
| - 12.5 $\mu$L Haloperoxidase | - 12.5 $\mu$L Haloperoxidase | |
| - a *Bacillus atrophaeus* spore disc ($10^6$ spores). | - a *Bacillus atrophaeus* spore disc ($10^6$ spores). | |
| - 50 $\mu$L Hydrogen peroxide | - 50 $\mu$L Hydrogen peroxide | |

The vials from Table 3 were incubated at 40°C for 30 minutes. To stop the reaction 500 $\mu$L Sodium thiosulphate was added, and incubated for 15 minutes at room temperature (approximately 23°C). Each disc was then transferred to a tube containing Tween 80 and glass beads, and the tubes were shaken for 15 minutes at 300 rpm.
After that a dilution series was made in MilliQ water and 500 $\mu$L from the dilutions $10^0$ to $10^{-3}$ were plated onto LB agar plates (14 cm plates). The plates were incubated for 48 hours at 37°C, and the average number of colony forming units (CFU) on each plate was registered (shown in Table 4).

Table 4.

| Dilution | Vial 1 | Vial 2 | Vial 3 (control) |
|---|---|---|---|
| $10^0$ (500 $\mu$L) | 10 | 3 | >> 200 |
| $10^{-1}$ (500 $\mu$L) | 1 | 0 | >> 200 |
| $10^{-2}$ (500 $\mu$L) | 0 | 0 | >> 200 |
| $10^{-3}$ (500 $\mu$L) | 0 | 0 | 90 |
| Calculated number of spores left on disc after treatment | 100 spores/disc | 30 spores/disc | 900,000 spores/disc |

The results shown in Table 4 indicate that the haloperoxidase solution of the invention has a clear and significant sporicidal effect, which is enhanced when the solution is used with a commercial cleaning agent.

The number of spores able to germinate after treatment with the haloperoxidase solution alone was reduced 4 log units. If the solution was used with a commercial cleaning agent, the number of spores able to germinate was reduced 4-5 log units.

**EXAMPLE 5**

Killing spores with combinations of chloride, bromide and ammonium ions

The following reagents were prepared:

- DMG buffer (DiMethylGlutamic acid, Sigma D4379), 100 mM, pH adjusted to 7.0 with NaOH;
- $1 \times 10^9$ *Bacillus atrophaeus* spores per mL (*Bacillus atrophaeus* spores: SUS-1-8 Log. Raven Labs, www. Ravenlabs.com);
- 40 mg/L Haloperoxidase from *Curvularia verrucolosa* (see WO 97/04102) in MilliQ water;
- 500 mM Sodium bromide (NaBr) in Milli Q water;
- 1000 mM Sodium chloride (NaCl) in MilliQ water;
- 500 mM Ammonium chloride ($NH_4Cl$) in MilliQ water

   10 mM Hydrogen peroxide ($H_2O_2$) in MilliQ water;
   5 % (w/v) Sodium thiosulphate ($Na_2S_2O_3$) in MilliQ water; and

- LB plates were made from 37 g LB Agar (Merck 0283) dissolved in 1000 mL water.

In vials, reagents were mixed according to Table 5.

Table 5.

| Reagent | 1 mM bromide + 24 mM chloride | 5 mM bromide + 24 mM chloride | 24 mM chloride |
|---|---|---|---|
| NaCl ($\mu$L) | 9 | 9 | 9 |
| NaBr ($\mu$L) | 1 | 5 | 0 |
| $NH_4Cl$ ($\mu$L) | 6 | 6 | 6 |
| DMG buffer pH 7.0 ($\mu$L) | 250 | 250 | 250 |
| Haloperoxidase ($\mu$L) | 100 | 100 | 100 |
| $H_2O_2$ ($\mu$L) | 100 | 100 | 100 |
| Spores ($\mu$L) | 5 | 5 | 5 |
| MilliQ water ($\mu$L) | 24 | 20 | 25 |

The reaction was started by adding hydrogen peroxide. A vial with 10 $\mu$L spore suspension and 490 $\mu$L MilliQ water acted as a control.
The vials were incubated at 40°C for 30 minutes. The reaction was then quenched by addition of 500 $\mu$L sodium thiosulphate, which was allowed to react for 15 minutes.
After that, a dilution series was made in MilliQ water, and 100 $\mu$L from the dilutions $10^0$ to $10^{-5}$ were plated (in duplicate) on LB agar plates. The plates were incubated for 48 hours at 33°C, and the average number of colony forming units per plate (CFU/plate) on each set of plates were registered (shown in Table 6).

Table 6.

| | Dilution | 1 mM bromide + 24 mM chloride | 5 mM bromide + 24 mM chloride | 24 mM chloride | Control |
|---|---|---|---|---|---|
| CFU/plate | $10^0$ | 31 | 28 | - | - |
| | $10^{-1}$ | 4 | 1 | 40 | - |
| | $10^{-2}$ | 0 | 0 | 3 | - |
| | $10^{-3}$ | 0 | 0 | 0 | - |
| | $10^{-4}$ | - | - | - | 180 |
| | $10^{-5}$ | - | - | - | 20 |
| CFU/mL | - | $3\times10^2$ | $3\times10^2$ | $4\times10^3$ | $2\times10^7$ |
| Kill (Log Units) | - | 4.8 | 4.8 | 3.7 | - |

The results shown in Table 6 indicate that addition of bromide to the Haloperoxidase/ chloride/ammonium solution boosts the sporicidal effect with at least 1 log unit.

The number of spores able to germinate after the treatment was reduced at least 1 log unit more than when only treated with the chloride/ammonium/haloperoxidase solution.

## EXAMPLE 6

Killing of *Bacillus* spores with varying ratios of bromide/chloride ions

The following reagents were prepared:

- DMG buffer (DiMethylGlutamic acid, Sigma D4379), 100 mM, pH adjusted to 7.0 with NaOH;
- $1\times10^9$ *Bacillus atrophaeus* spores per mL (*Bacillus atrophaeus* spores: SUS-1-8 Log. Raven Labs, www. Ravenlabs.com);
- 250 mg/L Haloperoxidase from *Curvularia verrucolosa* (see WO 97/04102) in MilliQ water;
- 50 mM Sodium bromide (NaBr) in MilliQ water;
- 200 mM Sodium chloride (NaCl) in MilliQ water;
- 200 mM Ammonium chloride ($NH_4Cl$) in MilliQ water
- 10 mM Hydrogen peroxide ($H_2O_2$) in MilliQ water;
- 5% (w/v) Sodium thiosulphate ($Na_2S_2O_3$) in MilliQ water; and
- LB plates were made from 37 g LB Agar (Merck 0283) dissolved in 1000 mL water.

In vials, reagents were mixed according to Table 7.

Table 7.

| Reagents | Bromide/chloride | | | | | |
|---|---|---|---|---|---|---|
| | 25 mM/25 mM | 5 mM/25 mM | 2.5 mM/25 mM | 1 mM/25 mM | 0.5 mM/25 mM | 0.25 mM/25 mM |
| NaCl ($\mu$L) | 47.5 | 47.5 | 47.5 | 47.5 | 47.5 | 47.5 |
| NaBr ($\mu$L) | 250 | 50 | 25 | 10 | 5 | 2.5 |
| $NH_4Cl$ ($\mu$L) | 15 | 15 | 15 | 15 | 15 | 15 |
| Buffer ($\mu$L) | 50 | 50 | 50 | 50 | 50 | 50 |
| Haloperoxidase ($\mu$L) | 16 | 16 | 16 | 16 | 16 | 16 |
| $H_2O_2$ ($\mu$L) | 100 | 100 | 100 | 100 | 100 | 100 |
| Spores ($\mu$L) | 10 | 10 | 10 | 10 | 10 | 10 |
| MilliQ water ($\mu$L) | 11.5 | 211.5 | 236.5 | 251.5 | 256.5 | 259 |

The reaction was started by adding hydrogen peroxide. A vial with 10 μL spore suspension and 490 μL MilliQ water acted as a control.

The vials were incubated at 22°C for 30 minutes. The reaction was then quenched by addition of 500 μL sodium thiosulphate, which was allowed to react for 15 minutes.

After that a dilution series was made in MilliQ water, and 100 μL from the dilutions $10^0$ to $10^{-5}$ were plated (in duplicate) onto LB agar plates. The plates were incubated for 48 hours at 33°C, and the average number of colony forming units per plate (CFU/plate) on each set of plates was registered (shown in Table 8).

Table 8.

| Dilution | Control | Bromide/chloride | | | | | |
|---|---|---|---|---|---|---|---|
| | | 25 mM/25 mM | 5 mM/25 mM | 2.5 mM/25 mM | 1 mM/25 mM | 0.5 mM/25 mM | 0.25 mM/25 mM |
| $10^0$ | - | - | - | - | - | - | - |
| $10^{-1}$ | - | 35 | 61 | 62 | 11 | 41 | 6 |
| $10^{-2}$ | - | - | - | - | - | - | - |
| $10^{-3}$ | - | - | 3 | 1 | 0 | 4 | 1 |
| $10^{-4}$ | - | - | - | - | - | - | - |
| $10^{-5}$ | 20 | - | - | - | - | - | - |
| CFU/mL | $2 \times 10^7$ | $3.5 \times 10^3$ | $5 \times 10^3$ | $6 \times 10^3$ | $1 \times 10^3$ | $4 \times 10^3$ | $6 \times 10^2$ |
| Kill (Log Units) | - | 3.7 | 3.6 | 3.5 | 4.3 | 3.7 | 4.4 |

The results indicate that the biocidal effect is more or less independent of the bromide concentration, but with a inclination of a slightly better kill with low bromide/chloride ratios.

## EXAMPLE 7

Killing of *Bacillus* spores in liquid preparations at various pH values

The following reagents were prepared:

- Phosphate buffer 100 mM, with the following pH values: pH 6.0, pH 6.5, pH 7.0, pH 7.4 and pH 8.0;
- Britton Robinson buffer 100 mM, pH 8.5;
- $1 \times 10^9$ *Bacillus atrophaeus* spores per mL (*Bacillus atrophaeus* spores: SUS-1-8 Log. Raven Labs, www. Ravenlabs.com);
- 250 mg/L Haloperoxidase from *Curvularia verrucolosa* (see WO 97/04102) in MilliQ water;
- 200 mM Sodium chloride (NaCl) in MilliQ water;
- 50 mM Sodium bromide (NaBr) in MilliQ water;
- 200 mM Ammonium chloride ($NH_4Cl$) in MilliQ water;
- 10 mM Hydrogen peroxide ($H_2O_2$) in MilliQ water; and
- LB plates were made from 37 g LB Agar (Merck 0283) dissolved in 1000 mL water.

In vials, reagents were mixed according to Table 9.

Table 9.

| Reagents | pH 6.0 | pH 6.5 | pH 7.0 | pH 7.4 | pH 8.0 | pH 8.5 |
|---|---|---|---|---|---|---|
| NaCl (μL) | 47.5 | 47.5 | 47.5 | 47.5 | 47.5 | 47.5 |
| NaBr (μL) | 5 | 5 | 5 | 5 | 5 | 5 |
| $NH_4Cl$ (μL) | 15 | 15 | 15 | 15 | 15 | 15 |
| Buffer (μL) | 50 | 50 | 50 | 50 | 50 | 50 |

(continued)

| Reagents | pH 6.0 | pH 6.5 | pH 7.0 | pH 7.4 | pH 8.0 | pH 8.5 |
|---|---|---|---|---|---|---|
| Haloperoxidase (μL) | 16 | 16 | 16 | 16 | 16 | 16 |
| $H_2O_2$ (μL) | 100 | 100 | 100 | 100 | 100 | 100 |
| Spore solution (μL) | 10 | 10 | 10 | 10 | 10 | 10 |
| MilliQ water (μL) | 256.5 | 256.5 | 256.5 | 256.5 | 256.5 | 256.5 |

The reaction was started by adding hydrogen peroxide. A vial with 10 μL spore suspension and 490 μL MilliQ water acted as a control.

The vials were incubated at either room temperature (approximately 22°C), at 40°C or at 50°C, for 30 minutes.

Then the reaction was quenched by addition of 500 μL sodium thiosulphate, which was allowed to react for 10 minutes.

After that, a dilution series was made in MilliQ water, and 100 μL from the dilutions $10^0$ to $10^{-5}$ were plated (in duplicate) on LB agar plates. The plates were incubated for 48 hours at 33°C, and the average number of colony forming units (CFU) on each pair of duplicate plates was registered (shown in Tables 10-12 below).

Table 10. Incubation temperature = 22°C. Average CFU at each dilution of spores treated with the haloperoxidase/chloride/ammonium solution.

| Dilution | pH 6.0 | pH 6.5 | pH 7.0 | pH 7.4 | pH 8.0 | pH 8.5 | Control |
|---|---|---|---|---|---|---|---|
| $10^0$ | - | 61 | 69 | 18 | - | - | - |
| $10^{-1}$ | - | 5 | 5 | 2 | $\sim 10^3$ | $\sim 10^5$ | - |
| $10^{-2}$ | - | 0 | - | - | - | - | - |
| $10^{-3}$ | - | 0 | - | - | - | - | - |
| $10^{-4}$ | - | - | - | - | - | - | 186 |
| $10^{-5}$ | - | - | - | - | - | - | 17 |

Table 11. Incubation temperature = 40°C. Average CFU at each dilution of spores treated with the haloperoxidase/chloride/ammonium solution.

| Dilution | pH 6.0 | pH 6.5 | pH 7.0 | pH 7.4 | pH 8.0 | pH 8.5 | Control |
|---|---|---|---|---|---|---|---|
| $10^0$ | - | - | - | - | - | - | - |
| $10^{-1}$ | - | 36 | 34 | 31 | - | - | - |
| $10^{-2}$ | - | 0 | - | - | - | - | - |
| $10^{-3}$ | 5 | 0 | - | - | 28 | - | - |
| $10^{-4}$ | 9 | - | - | - | 7 | 14 | 238 |
| $10^{-5}$ | - | - | - | - | - | 5 | 32 |

Table 12. Incubation temperature = 50°C. Average CFU at each dilution of spores treated with the haloperoxidase/chloride/ammonium solution.

| Dilution | pH 6.0 | pH 6.5 | pH 7.0 | pH 7.4 | pH 8.0 | pH8.5 | Control |
|---|---|---|---|---|---|---|---|
| $10^0$ | - | - | - | - | - | - | - |
| $10^{-1}$ | - | 53 | 48 | 600 | - | - | - |
| $10^{-2}$ | 6 | - | - | - | - | - | - |
| $10^{-3}$ | - | - | - | - | 14 | - | - |

(continued)

| Dilution | pH 6.0 | pH 6.5 | pH 7.0 | pH 7.4 | pH 8.0 | pH8.5 | Control |
|---|---|---|---|---|---|---|---|
| $10^{-4}$ | - | - | - | - | - | 9 | - |
| $10^{-5}$ | - | - | - | - | - | - | 36 |

The results are summarized in Table 13 below.

Table 13.

| Temp (°C) | | pH 6.0 | pH 6.5 | pH 7.0 | pH 7.4 | pH 8.0 | pH 8.5 | Control |
|---|---|---|---|---|---|---|---|---|
| 22 | CFU/mL | - | $6 \times 10^2$ | $7 \times 10^2$ | $2 \times 10^2$ | $1 \times 10^5$ | $1 \times 10^7$ | $2 \times 10^7$ |
| | Kill (Log Units) | - | 4.5 | 4.5 | 5 | 2.3 | 0.3 | - |
| 40 | CFU/mL | $5 \times 10^4$ | $4 \times 10^3$ | $3 \times 10^3$ | $3 \times 10^3$ | $3 \times 10^5$ | $5 \times 10^6$ | $2 \times 10^7$ |
| | Kill (Log Units) | 2.6 | 3.7 | 3.8 | 3.8 | 1.8 | 0.6 | - |
| 50 | CFU/mL | $6 \times 10^3$ | $5 \times 10^3$ | $5 \times 10^3$ | $6 \times 10^4$ | $1.5 \times 10 5$ | $9 \times 10^5$ | $3 \times 10^7$ |
| | Kill (Log Units) | 3.7 | 3.8 | 3.8 | 2.7 | 2.3 | 1.5 | - |

The results shown in Table 13 indicate that the haloperoxidase / chloride / bromide / ammonium solution has a clear and significant sporicidal effect. The effect is at its maximum in the pH range 6.5 - 7.4 at all 3 tested temperatures.

**EXAMPLE 8**

Killing of spores at varying $H_2O_2$ concentrations

The following reagents were prepared:

- DMG buffer (DiMethylGlutamic acid, Sigma D4379), 100 mM, pH adjusted to 7.0 with NaOH;
- $1 \times 10^9$ *Bacillus atrophaeus* spores per mL (*Bacillus atrophaeus* spores: SUS-1-8 Log. Raven Labs, www. Ravenlabs.com);
- 250 mg/L Haloperoxidase from *Curvularia verrucolosa* (see WO 97/04102) in MilliQ water;
- 50 mM Sodium bromide (NaBr) in MilliQ water;
- 200 mM Sodium chloride (NaCl) in MilliQ water;
- 200 mM Ammonium chloride ($NH_4Cl$) in MilliQ water
- 25 mM Hydrogen peroxide ($H_2O_2$) in MilliQ water;
- 1 % (w/v) Sodium thiosulphate ($Na_2S_2O_3$) in MilliQ water; and
- LB plates were made from 37 g LB Agar (Merck 0283) dissolved in 1000 mL water.

In vials, reagents were mixed according to Table 14.

Table 14.

| Reagents | 0.1 mM $H_2O_2$ | 0.5 mM $H_2O_2$ | 1 mM $H_2O_2$ | 2 mM $H_2O_2$ | 5 mM $H_2O_2$ | 10 mM $H_2O_2$ |
|---|---|---|---|---|---|---|
| NaCl ($\mu$L) | 47.5 | 47.5 | 47.5 | 47.5 | 47.5 | 47.5 |
| NaBr ($\mu$L) | 5 | 5 | 5 | 5 | 5 | 5 |
| $NH_4Cl$ ($\mu$L) | 15 | 15 | 15 | 15 | 15 | 15 |
| Buffer ($\mu$L) | 50 | 50 | 50 | 50 | 50 | 50 |
| Haloperoxidase ($\mu$L) | 16 | 16 | 16 | 16 | 16 | 16 |
| $H_2O_2$ ($\mu$L) | 2 | 10 | 20 | 40 | 100 | 150 |
| Spore solution ($\mu$L) | 10 | 10 | 10 | 10 | 10 | 10 |
| MilliQ water ($\mu$L) | 354.5 | 346.5 | 336.5 | 316.5 | 256.5 | 206.5 |

The reaction was started by adding hydrogen peroxide. A vial with 10 μL spore suspension and 490 μL MilliQ water acted as a control.

The vials were incubated at room temperature (approximately 23°C) for 30 minutes. The reaction was quenched by addition of 500 μL sodium thiosulphate, which was allowed to react for 10 minutes at room temperature.

After that, a dilution series was made in MilliQ water, and 100 μL from the dilutions $10^0$ to $10^{-5}$ were plated (in duplicate) on LB agar plates. The plates were incubated for 48 hours at 33°C, and the average number of colony forming units /plate (CFU/plate) on each pair of duplicate plates was registered (shown in Table 15).

Table 15.

| Dilution | Control | 0.1 mM $H_2O_2$ | 0.5 mM $H_2O_2$ | 1 mM $H_2O_2$ | 2mM $H_2O_2$ | 5 mM $H_2O_2$ | 10 mM $H_2O_2$ |
|---|---|---|---|---|---|---|---|
| $10^{-1}$ | - | - | 150 | 2 | 0 | 1 | 3 |
| $10^{-2}$ | - | - | - | 1 | 0 | 1 | 1 |
| $10^{-3}$ | - | 800 | - | 0 | 0 | 0 | 0 |
| $10^{-4}$ | 138 | - | - | 0 | 0 | 0 | 0 |
| $10^{-5}$ | 23 | - | - | 0 | 0 | 0 | 0 |
| CFU/mL | $2\times10^7$ | $8\times10^6$ | $1.5\times10^6$ | $5\times10^2$ | 5 | $1\times10^2$ | $3\times10^2$ |
| Kill (Log Units) | - | 0.4 | 1.1 | 4.6 | 6.6 | 5.3 | 4.8 |

The results in Table 15 shows that the highest kill is achieved with 2-5 mM $H_2O_2$, indicating that this is the optimal hydrogen peroxide concentration under these experimental conditions. All concentrations in the range 1 mM - 10 mM gives acceptable decontamination performance.

**EXAMPLE 9**

Killing of spores at varying ratios of chloride/bromide/ammonium; constant haloperoxidase concentration

The following reagents were prepared:

- DMG buffer (DiMethylGlutamic acid, Sigma D4379), 100 mM, pH adjusted to 7.0 with NaOH;
- $1\times10^9$ *Bacillus atrophaeus* spores per mL (*Bacillus atrophaeus* spores: SUS-1-8 Log. Raven Labs, www. Ravenlabs.com);
- 250 mg/L Haloperoxidase from *Curvularia verrucolosa* (see WO 97/04102) in MilliQ water;
- 50 mM Sodium bromide (NaBr) in MilliQ water;
- 200 mM Sodium chloride (NaCl) in MilliQ water;
- 500 mM Sodium chloride (NaCl) in MilliQ water;
- 200 mM Ammonium chloride ($NH_4Cl$) in MilliQ water
- 20 mM Ammonium sulphate (($NH_4$)$_2SO_4$) in MilliQ water
- 100 mM Ammonium sulphate (($NH_4$)$_2SO_4$) in MilliQ water
- 25 mM Hydrogen peroxide ($H_2O_2$) in MilliQ water;
- 1 % (w/v) Sodium thiosulphate ($Na_2S_2O_3$) in MilliQ water; and
- LB plates were made from 37 g LB Agar (Merck 0283) dissolved in 1000 mL water.

Following the methods and principles of the previous Examples, 500 μL reaction mixture was made using the above-mentioned solutions. The spore treatments were carried out in vials containing:

- 0 mM / 0.5 mM / 1 mM / 5 mM bromide (Br⁻);
- 0 mM / 25 mM / 50 mM / 66.7 mM / 100 mM / 150 mM chloride (Cl⁻); and
- 0 mM / 0.1 mM / 1 mM / 5 mM / 10 mM / 16.7 mM / 25 mM /50 mM / 100 mM ammonium ($NH_4^+$)

in various combinations; and

- 8 ppm Haloperoxidase;
- 2 mM $H_2O_2$;
- $2\times10^7$ spores;

- 10 mM DMG buffer; and
- MilliQ water ad 500μL

The reaction was started by adding hydrogen peroxide. A vial with 10 μL spore suspension and 490 μL MilliQ water acted as a control.

The vials were incubated at room temperature (approximately 23°C) for 30 minutes. The reaction was quenched by addition of 500 μL sodium thiosulphate, which was allowed to act for 10 minutes at room temperature.

After that, a dilution series was made in MilliQ water, and 100 μL from the dilutions $10^0$to$10^5$were plated (in duplicate) on LB agar plates. The plates were incubated for 48 hours at 33°C, and the average number of colony forming units/plate (CFU/plate) on each set of plates was registered.

Using the CFU/mL values, the spore kill in log units was calculated, and the results are shown in Table 16.

Table 16. Spore kill in Log Units

| | | 0 mM bromide | 0.1 mM bromide | 1 mM bromide | 5 mM bromide |
|---|---|---|---|---|---|
| 0 mM chloride | 0 mM ammonium | - | 1.2 | 1.3 | 0.1 |
| | 0.1 mM ammonium | - | 1.7 | 0.5 | - |
| | 1 mM ammonium | - | 2.5 | 3.4 | - |
| | 5 mM ammonium | - | 1.4 | 5.8 | 4.2 |
| | 16.6 mM ammonium | - | 0.6 | 5.2 | 3.3 |
| | 25 mM ammonium | - | 0.3 | 5 | 4.2 |
| 25 mM chloride | 0 mM ammonium | 0.2 | 0.7 | 0.6 | 0.1 |
| | 0.1 mM ammonium | 0.2 | - | - | - |
| | 1 mM ammonium | 0.5 | 4.8 | 3.5 | 3.1 |
| | 5 mM ammonium | 0.2 | 3.7 | 3.7 | 3.2 |
| | 10 mM ammonium | 0.2 | 1.8 | 4.2 | 3.8 |
| | 16.6 mM ammonium | 0.2 | 0.9 | 4.6 | 3.6 |
| | 25 mM ammonium | 0.2 | 1 | 5.5 | 4.5 |
| 50 mM chloride | 0 mM ammonium | 0.2 | 0.1 | 0.2 | 0.1 |
| | 0.1 mM ammonium | 0.3 | 0.4 | 0.5 | 0.2 |
| | 1 mM ammonium | 0.6 | 2.4 | 2.5 | 1.2 |
| | 5 mM ammonium | 0.4 | 2.5 | 4.2 | 4 |
| | 16.6 mM ammonium | 0.3 | 0.7 | 4.5 | 3.7 |
| | 50 mM ammonium | 0.3 | 0.3 | 3.5 | 4.2 |
| 66.7 mM chloride | 0 mM ammonium | 0.2 | - | - | - |
| | 0.1 mM ammonium | - | 0.5 | 0.5 | 0.5 |
| | 1 mM ammonium | - | 4.3 | 2.9 | 1.4 |
| | 5 mM ammonium | 0.3 | 1.8 | 5.2 | 3.2 |
| | 100 mM ammonium | 0.3 | 0.4 | 4.2 | 4.9 |
| 100 mM chloride | 0 mM ammonium | 0.2 | 0.1 | 1.2 | 0.1 |
| | 0.1 mM ammonium | - | 0.2 | 0.3 | 0.3 |
| | 1 mM ammonium | - | 2.9 | 2.7 | 1.1 |
| | 10 mM ammonium | 0.4 | 2.1 | 4.5 | 4.2 |
| | 100 mM ammonium | 0.2 | 0.4 | 4.2 | 4.9 |

(continued)

|  |  | 0 mM bromide | 0.1 mM bromide | 1 mM bromide | 5 mM bromide |
|---|---|---|---|---|---|
| 150 mM chloride | 0 mM ammonium | 0.2 | 0.4 | 0.4 | 0.4 |
|  | 0.1 mM ammonium | - | 0.3 | 0.4 | 0.4 |
|  | 1 mM ammonium | - | 2.9 | 1.5 | 1 |
|  | 5 mM ammonium | - | 1.9 | 2 | 1.7 |
|  | 10 mM ammonium | 0.6 | - | - | - |
|  | 50 mM ammonium | 0.2 | 1 | 3.9 | 3 |

The ratio of chloride / bromide / ammonium influences performance; generally a molar ratio of bromide/ammonium of 1/10 gives the highest kill.

The lower the chloride concentration is, the lower concentrations of bromide and ammonium are needed for at satisfactory spore kill.

The best spore killing performance was obtained with:

[chloride] $\leq$ 25 mM;

0.1 mM $\leq$ [bromide] $\leq$ 1 mM; and
5 mM $\leq$ [ammonium] $\leq$ 25 mM.

**EXAMPLE 10**

Killing of spores at varying ratios of chloride / bromide / ammonium; and at varying haloperoxidase / hydrogen peroxide concentration

The following reagents were prepared:

- DMG buffer (DiMethylGlutamic acid, Sigma D4379), 100 mM, pH adjusted to 7.0 with NaOH;
- 1x10$^9$ *Bacillus atrophaeus* spores per mL (*Bacillus atrophaeus* spores: SUS-1-8 Log. Raven Labs, www. Ravenlabs.com);
- 250 mg/L Haloperoxidase from *Curvularia verrucolosa* (see WO 97/04102) in MilliQ water;
- 50 mM Sodium bromide (NaBr) in MilliQ water;
- 500 mM Sodium chloride (NaCl) in MilliQ water;
- 200 mM Ammonium chloride (NH$_4$Cl) in MilliQ water;
- 25 mM Hydrogen peroxide (H$_2$O$_2$) in MilliQ water;
- 1 % (w/v) Sodium thiosulphate (Na$_2$S$_2$O$_3$) in MilliQ water; and
- LB plates were made from 37 g LB Agar (Merck 0283) dissolved in 1000 mL water.

Following the methods and principles of the previous Examples, 500 $\mu$L reaction mixture was made using the above mentioned solutions. The reactions were carried out in vials. The final concentrations of the constituents are as shown in Table 17.

Table 17.

| Haloperoxidase (ppm) | H$_2$O$_2$ (mM) | NaCl (mM) | NH$_4$Cl (mM) | NaBr (mM) | Vial No. |
|---|---|---|---|---|---|
| 8 | 2 | 0 | 0 | 0 | 1 |
| | | 0 | 0 | 2 | 2 |
| | | 5 | 5 | 0 | 3 |
| | | 5 | 5 | 2 | 4 |
| | | 10 | 2.5 | 1 | 5 |
| | | 15 | 0 | 0 | 6 |
| | | 15 | 0 | 2 | 7 |
| | | 20 | 5 | 0 | 8 |
| | | 20 | 5 | 2 | 9 |
| 20 | 5 | 2.5 | 2.5 | 1 | 10 |
| | | 7.5 | 0 | 1 | 11 |
| | | 10 | 2.5 | 1 | 12 |
| | | 10 | 2.5 | 0 | 13 |
| | | 10 | 2.5 | 1 | 14 |
| | | 10 | 2.5 | 2 | 15 |
| | | 12.5 | 5 | 1 | 16 |
| | | 17.5 | 2.5 | 1 | 17 |
| 32 | 8 | 0 | 0 | 0 | 18 |
| | | 0 | 0 | 2 | 19 |
| | | 5 | 5 | 0 | 20 |
| | | 5 | 5 | 2 | 21 |
| | | 10 | 2.5 | 1 | 22 |
| | | 15 | 0 | 2 | 23 |
| | | 15 | 0 | 0 | 24 |
| | | 20 | 5 | 2 | 25 |
| | | 20 | 5 | 0 | 26 |
| 2x10$^7$ spores | | | | | |
| 10 mM DMG buffer | | | | | |
| MilliQ water ad 500 $\mu$L | | | | | |

The ratio of haloperoxidase / hydrogen peroxide was the same that was found to be optimal in Example 6. The reaction was started with addition of hydrogen peroxide. A vial with 10 $\mu$L spore suspension and 490 $\mu$L MilliQ water acted as a control.

The vials were incubated at 60°C for 30 minutes. The reaction was quenched by addition of 500 $\mu$L sodium thiosulphate, which was allowed to react for 10 minutes at room temperature.

After that, a dilution series was made in MilliQ water, and 100 $\mu$L from the dilutions 10$^0$ to 10$^5$ were plated (in duplicate) on LB agar plates. The plates were incubated for 48 hours at 33°C, and the average number of colony forming units/plate (CFU/plate) on each set of plates was registered.

Using the CFU/mL values, the spore kill in log units was calculated, and the results are shown in Table 18.

Table 18.

| Haloperoxidase (ppm) | chloride (mM) | ammonium (mM) | Kill in Log Units | | |
|---|---|---|---|---|---|
| | | | 0 mM bromide | 1 mM bromide | 2 mM bromide |
| 8 | 0 | 0 | 0 | - | 0.7 |
| 8 | 5 | 5 | 1.6 | - | 3.4 |
| 8 | 10 | 2.5 | - | 3.5 | - |
| 8 | 15 | 0 | 3 | - | 0.4 |
| 8 | 20 | 5 | 2.4 | - | 3.6 |
| 20 | 2.5 | 2.5 | - | 3.8 | - |
| 20 | 7.5 | 0 | - | 0.5 | - |
| 20 | 10 | 2.5 | 3.6 | 3.3 | 3.6 |
| 20 | 10 | 5 | - | 3.6 | - |
| 20 | 12.5 | 5 | 3.3 | - | - |
| 20 | 17.5 | 2.5 | - | 3.6 | - |
| 32 | 0 | 0 | 0 | - | 0.1 |
| 32 | 5 | 5 | 4.6 | - | 4.7 |
| 32 | 10 | 2.5 | - | 4.8 | - |
| 32 | 15 | 0 | 2.2 | - | 0.4 |
| 32 | 20 | 5 | 3.1 | - | 3.8 |

The best spore killing performance was obtained with:

[haloperoxidase] = 32 ppm;

5 mM ≤ [chloride] ≤ 20 mM;
0 mM ≤ [bromide] ≤ 2 mM; and
2.5 mM ≤ [ammonium] ≤ 5 mM.

**EXAMPLE 11**

Fungal spore production

*Trichophyton mentagrophytes* ATCC 9233 was cultured on MEA plates and *Aspergillus niger*ATCC 9642 was grown on MEA slants.

The MEA culture medium was made as follows with deionized water: 30 g/L Malt extract, 3 g/L Soja peptone (papaic digest or soybean meal), agar 15 g/L, pH unadjusted.

*Trichophyton mentagrophytes* was inoculated at the centre of a MEA Petri dish and incubated at 30°C for 10 - 12 days.

The plate was then flooded with M9 buffer with 0.02 % Tween 80 and the spores were made into suspension by gently working the mycelial matt with a Drigalski spatula. The cell suspension was then filtered through Miracloth to remove hyphae and the spore number was subsequently determined by counting in a haemocytometer. This spore suspension was used in the experiments.

A slant with vigorous sporulating *A. niger* was harvested with M9 + 0.02 % Tween and the spore containing liquid filtered through sterile Miracloth to remove hyphae.

The composition of M9 is as follows: In milliQ water is dissolved 8.77 g/L disodium hydrogen phosphate ($Na_2HPO$, $2 H_2O$), 3 g/L potassium dihydrogen phosphate ($KH_2PO_4$), 4 g/L sodium chloride (NaCl), 0.2 g/L magnesium sulphate ($MgSO_4$, $7 H_2O$).

The spore number was subsequently determined by counting in a haemocytometer, and the spore suspension was adjusted to approx.$1 \times 10^7$ spores/mL. This spore suspension was used in the Examples. The spore suspensions were stored at 4°C for a maximum of 4 days.

**EXAMPLE 12**

Fungal spore kill with system freshly prepared fungal spores

The following reagents were prepared:

- Spore suspension of Aspergillus *niger* ATCC 9642, $1 \times 10^7$ spores/mL;
- Spore suspension of *Trichophyton mentagrophytes* ATCC 9233, $1 \times 10^7$ spores/mL;
- DMG buffer (DiMethylGlutamic acid, Sigma D4379), 100 mM, pH adjusted to 7.0 with NaOH;
- 100 mg/L Haloperoxidase from *Curvularia verrucolosa* (see WO 97/04102) in MilliQ water;
- 50 mM Sodium bromide (NaBr) in MilliQ water;
- 200 mM Sodium chloride (NaCl) in MilliQ water;
- 200 mM Ammonium chloride ($NH_4Cl$) in MilliQ water;
- 10 mM Sodium percarbonate ($2Na_2CO_3 \cdot 3(H_2O_2)$) in MilliQ water; and
- YPD plates were made from: 10 g Yeast extract, 20 g Peptone, 20 g Dextrose, 20 g agar, dissolved in 1000 mL water, pH unadjusted.

In vials the reagents were mixed according to Table 19. The spores used were all freshly made (i.e. not stored at 4°C for any period of time).

Table 19.

| Reagents | *T. mentagrophytes* | | *A. niger* | |
|---|---|---|---|---|
| | μL | mM, final | μL | mM, final |
| NaCl | 47.5 | 19 | 47.5 | 19 |
| NaBr | 5 | 0.5 | 5 | 0.5 |
| $NH_4Cl$ | 15 | 6 | 15 | 6 |
| DMG buffer | 125 | 25 | 125 | 25 |
| Haloperoxidase | 40 | 8 ppm | 40 | 8 ppm |
| Sodium Percarbonate | 33.5 | 0.67 | 33.5 | 0.67 |
| Spore suspension | 50 | $1 \times 10^6$ | 50 | $1 \times 10^6$ |
| $H_2O$ | 184 | - | 184 | - |
| Final volume | 500 | - | 500 | - |

All reagents, except percarbonate, were mixed in a 1.8 mL NUNC cryotube; the spores were added and the experiment started by adding the percarbonate solution.
The tubes were incubated at 40°C (in a thermo block) for 30 minutes. After 30 minutes incubation, 500 μL sterile water was added to each tube and 10-fold dilution series were made.
200 μL from the undiluted sample and 100 μL from each of the dilutions were plated on YPD plates. The plates were incubated at 30°C for 2 - 4 days and CFU/mL calculated.
50 μL spore suspension added to 450 μL sterile water with subsequent dilution series, plating 100 μL/plate and determination of CFU/mL, acted as growth control.
*Aspergillus* spores are hydrophobic, and it was difficult to get the spores in a homogenous suspension.
Table 20 shows the recorded CFU/plate and the calculated CFU/ml, as well as the spore kill (Log Units).

Table 20.

| Dilution | *Trichophyton mentagrophytes* | | *Aspergillus niger* | | *Trichophyton* control | | *Aspergillus* control | |
|---|---|---|---|---|---|---|---|---|
| $10^0$ | 0 | 0 | 3 | 15 | - | - | - | - |
| $10^{-1}$ | 0 | 0 | 0 | 0 | - | - | - | - |

(continued)

| Dilution | Trichophyton mentagrophytes | | Aspergillus niger | | Trichophyton control | | Aspergillus control | |
|---|---|---|---|---|---|---|---|---|
| $10^{-2}$ | 0 | 0 | 0 | 0 | - | - | - | - |
| $10^{-3}$ | - | - | - | - | 7 | 13 | 15 | 12 |
| $10^{-4}$ | - | - | - | - | 2 | 3 | 1 | 1 |
| $10^{-5}$ | - | - | - | - | - | - | - | - |
| CFU/mL | 0 | | $9\times10^1$ | | $2\times10^5$ | | $1\times10^5$ | |
| Kill (Log Units) | 5 | | 3 | | - | | - | |

The *Trichophyton* spores were completely inactivated, whereas treatment of the *Aspergillus* spores resulted in 3 log units reduction in the number of viable spores.

**EXAMPLE 13**

Fungal spore kill on stored fungal spores

The following reagents were prepared:

- Spore suspension of *Aspergillus niger ATCC9642,* $1\times10^7$ spores/mL, stored for 2 days at 4°C;
- Spore suspension of *Trichophyton mentagrophytes* ATCC9233, $1\times10^7$ spores/mL, stored for 2 days at 4°C;
- DMG buffer (DiMethylGlutamic acid, Sigma D4379), 100 mM, pH adjusted to 7.0 with NaOH;
- 100 mg/L Haloperoxidase from *Curvularia verrucolosa* (see WO 97/04102) in MilliQ water;
- 50 mM Sodium bromide (NaBr) in Milli Q water;
- 200 mM Sodium chloride (NaCl) in MilliQ water;
- 200 mM Ammonium chloride ($NH_4Cl$) in MilliQ water;
- 10 mM sodium percarbonate ($2Na_2CO_3\cdot3(H_2O_2)$) in MilliQ water; and
- YPD plates were made from: 10g Yeast extract, 20 g Peptone, 20 g Dextrose, 20g agar, dissolved in 1000 mL water, pH unadjusted.

In vials, reagents were mixed according to Table 21.

Table 21.

| Reagents | T. mentagrophytes | | A. niger | |
|---|---|---|---|---|
| | μL | mM, final | μL | mM, final |
| NaCl | 142.5 | 19 | 142.5 | 19 |
| NaBr | 15 | 0.5 | 15 | 0.5 |
| $NH_4Cl$ | 45 | 6 | 45 | 6 |
| DMG buffer | 375 | 25 | 375 | 25 |
| Enzyme | 120 | 8 ppm | 120 | 8 ppm |
| Sodium Percarbonate | 100.5 | 0.67 | 100.5 | 0.67 |
| Spores | 150 | $1\times10^6$ | 150 | $1\times10^6$ |
| $H_2O$ | 552 | - | 552 | - |
| Final volume | 1500 | - | 1500 | - |

All ingredients, except percarbonate, were mixed in an 1.8 mL NUNC cryotube, the spores were added and the experiment started by adding the percarbonate solution.
The tubes were incubated at 40°C (in a thermo block) for 30 minutes. After 30 minutes incubation a 10-fold dilution series was made.

333 μL from each dilution were plated on YPD plates. The plates were incubated at 30°C for 2 - 4 days and CFU/mL calculated.

50 μL spore suspension added to 450 μL sterile water with subsequent dilution series, plating 100 μL/plate and determination of CFU/mL, acted as growth control.

Table 22 shows the recorded CFU/plate and the calculated CFU/ml, as well as the spore kill (Log Units).

Table 22.

| Dilution | *Trichophyton mentagrophytes* | | *Aspergillus niger* | | | *Trichophyton* growth control | | *Aspergillus* growth control | |
|---|---|---|---|---|---|---|---|---|---|
| $10^0$ | 0 | 0 | 0 | 0 | 0 | - | - | - | - |
| $10^{-1}$ | 0 | 0 | 0 | 0 | 0 | - | - | - | - |
| $10^{-2}$ | 0 | 0 | 0 | 0 | 0 | - | - | - | - |
| $10^{-3}$ | - | - | | - | - | - | - | - | - |
| $10^{-4}$ | - | - | - | - | - | 6 | 3 | 3 | 3 |
| $10^{-4}$ | - | - | - | - | - | 0 | 1 | 0 | 1 |
| CFU/mL | 0 | | 0 | | | $5\times10^5$ | | $5\times10^5$ | |
| Kill (Log Units) | 5 | | 5 | | | - | | - | |

Treatment of both *Trichophyton* and *Aspergillus* spores resulted in at least 5 log units reduction in the number of viable spores.

## EXAMPLE 14

Killing of *Bacillius subtilis* spores on polyester sutures

The following reagents were prepared:

- DMG buffer (DiMethylGlutamic acid, Sigma D4379), 50 mM, pH adjusted to 7.0 with NaOH;
- Phosphate buffer, 100mM, pH adjusted to 7.0 with NaOH;
- 200 mg/L Haloperoxidase from *Curvularia verrucolosa* (see WO 97/04102) in 50 mM DMG buffer;
- 400 mM Sodium chloride (NaCl) in MilliQ water;
- 200 mM Ammonium chloride ($NH_4Cl$) in MilliQ water;
- 40 mM Hydrogen peroxide ($H_2O_2$) in MilliQ water;
- 10% Sodium thiosulphate ($Na_2S_2O_3$);
- Synthetic hard water at 40°dH; and
- 200x dilution of a commercial non-ionic surfactant.

Other materials:

- tubes containing 5 mL of 1% Tween 80 in water and approximately 1 mL glass beads (3 mm diameter);
- LB plates were made from 37 g LB Agar (Merck 0283) dissolved in 1000 mL water;
- Nunc Cryo Tubes;
- Adjustable heating block; and
- Polyester sutures coated with $10^6$ *Bacillius subtilis* ATCC 19659 spores (Presque Isle Cultures, catalog # SP-BS) were used for the experiment (one suture was used for one treatment).

In a Nunc Cryo tube the following components were mixed:

50 μL Phosphate buffer,
12.5 μL NaCl solution,

25 $\mu$L NH$_4$Cl solution,
160 $\mu$L Haloperoxidase solution,
500 $\mu$L Synthetic hard water 40°dH,
15.4 $\mu$L 200x diluted non ionic surfactant,
37.1 $\mu$L MilliQ water, and

One *Bacillus subtilis* spore coated polyester suture (10$^6$ spores).

A negative control was prepared as above, but with the haloperoxidase solution substituted with MilliQ water.

The reactions were subsequently started by the addition of 200 $\mu$L Hydrogen peroxide. The vials were incubated at 40°C for 20 minutes. To stop the reaction 500 $\mu$L Sodium thiosulphate was added, and incubated for 10 minutes at room temperature (approximately 23°C). Each suture was then transferred to a tube containing 1% Tween 80 in water and glass beads, and the tubes were shaken for 15 minutes at 300 rpm to recover the remaining spores.

After that a dilution series was made in MilliQ water, and 100 $\mu$L from the dilutions 10$^0$ to 10$^{-3}$ were plated on LB agar plates (14 cm plates). The plates were incubated for 48 hours at 37°C, and the average number of colony forming units (CFU) on each plate was registered. The number of colonies was used to calculate the log reduction (kill) in number of recoverable bacteria, which is shown in Table 23.

Table 23.

|  | Log reduction (Log Units) |
| --- | --- |
| Negative control | 0 |
| Treated | > 6 |
| Number of recovered spores from control suture | 1.5x10$^6$ spores/suture |

The result shown in Table 23 demonstrates that the haloperoxidase solution of the invention has a clear and significant sporicidal effect effect compared to a negative control. The number of spores able to germinate after the treatment with the haloperoxidase was reduced 6 log units in 90 min.

## EXAMPLE 15

Disinfection of *Bacillus atrophaeus* spores on a stainless steel carrier

The following reagents were prepared:

- DMG buffer (DiMethylGlutamic acid, Sigma D4379), 50 mM, pH adjusted to 7.0 with NaOH;
- 200 mg/L Haloperoxidase from *Curvularia verrucolosa* (see WO 97/04102) in 50 mM DMG buffer;
- 400 mM Sodium chloride (NaCl) in MilliQ water;
- 100 mM Ammonium sulphate (($NH_4$)$_2$SO$_4$) in MilliQ water;
- 10 mM Hydrogen peroxide (H$_2$O$_2$) in MilliQ water;
- 50 mM Sodium bromide (NaBr) in MilliQ water; and
- 5% Sodium thiosulphate (Na$_2$S$_2$O$_3$).

Other materials:

- tubes containing 5 mL of 1% Tween 80 in water and approximately 1 mL glass beads (3 mm diameter);
- LB plates were made from 37 g LB Agar (Merck 0283) dissolved in 1000 mL water;
- Nunc Cryo Tube;
- Adjustable heating block; and 10$^6$
- Stainless steel discs each containing 10$^6$ *Bacillus atrophaeus* spores (Raven Labs cat. # 1-6100ST, ATCC 9372) were used for the experiment (one disc was used for one treatment).

In a Nunc Cryo tube the following components were mixed:

478 µL DMG buffer,

7.5 µL NaBr (Substituted with DMG buffer in experiment without NaBr) 47 µL NaCl solution,

38 µL $(NH_4)_2SO_4$ solution,

30 µL Haloperoxidase solution, and

One *Bacillus atrophaeus* spore disc ($10^6$ spores).

To test the effect of sodium bromide on the kill efficacy experiments were done both with and without NaBr. In the latter experiments NaBr was substituted with DMG buffer.

A negative control was prepared as above but with the haloperoxidase solution substituted with DMG buffer.

The reactions were subsequently started by the addition of 150 µL Hydrogen peroxide.

The vials were incubated at 40°C for 20 minutes. To stop the reaction 750 µL Sodium thiosulphate was added, and incubated for 10 minutes at room temperature (approximately 23°C). Each disc was then transferred to a tube containing 1% Tween 80 in water and glass beads, and the tubes were shaken for 15 minutes at 300 rpm to recover the remaining spores.

After that a dilution series was made in MilliQ water, and 100 µL from the dilutions $10^0$ to $10^{-3}$ were plated on LB agar plates (14 cm plates). The plates were incubated for 48 hours at 37°C, and the average number of colony forming units (CFU) on each plate was registered.

The number of colonies was used to calculate the log reduction (kill) in number of recoverable bacteria, which is shown in Table 24.

Table 24.

|  | Log reduction (Log Units) |
|---|---|
| Negative control | 0 |
| Treated (without NaBr) | 1 |
| Treated (with NaBr) | > 3 |
| Number of recovered spores from control cylinder | $10^5$ spores/suture |

**EXAMPLE 16, Washer Disinfector tests**

Killing of spores on polyester sutures and porcelain cylinders in a Washer Disinfector

The following reagents were prepared:

- Phosphate buffer, 1 M, pH adjusted to 7.0 with NaOH;
- 64 mg/mL Haloperoxidase from *Curvularia verrucolosa* (see WO 97/04102) in water;
- 5 M Sodium chloride (NaCl) in MilliQ water;
- 4 M Ammonium chloride ($NH_4Cl$) in MilliQ water;
- 9.8 M (30%) Hydrogen peroxide ($H_2O_2$) in MilliQ water;
- commercial non-ionic surfactant (BASF LF900).

Other materials:

- tubes containing 5 mL of 1% Tween 80 in water and approximately 1 mL glass beads (3 mm diameter);
- LB plates (14 cm) were made from 37 g LB Agar (Merck 0283) dissolved in 1000 mL water;
- Polyester sutures coated with $10^6$ *Bacillius subtilis* ATCC 19659 spores (Presque Isle Cultures, catalog # SP-BS);
- Porcelain cylinders coated with $10^6$ *Bacillius subtilis* ATCC 19659 spores;
- Washer Disinfector, which carried out the following sequential steps:

  Pre-rinse cold water (2 min.) - Washing (22 min.) - Draining (1 min.) - Rinse (2 min.) - Draining (1 min.) - Rinse (2 min.) - Draining (1 min.) - Haloperoxidase addition and sporicidal treatment - Draining (1 min.) - Rinse (2 min.) - Draining (1 min.) - Rinse (2 min.) - Draining (1 min.) - End.

The washer disinfector used in the experiments was of the type "46-4", which is available from Getinge Aktiebolag, Getinge, Sweden. The standard washer disinfector of the type 46-4 was modified in that sense that it was provided with a feeding hopper connected to a hose, the hose ending inside the washing chamber of the washer disinfector. This made it possible to feed the chemicals of the "Haloperoxidase addition and sporicidal treatment"-step, as outlined below, directly into the washing chamber.

During the "Washing"-step the temperature was ramped up from approx. 20°C to approx. 60°C. The "Rinse"-steps were carried out at approx. 50°C. During the "Haloperoxidase addition and sporicidal treatment"-step, the following components were added in the Washer Disinfector (total volume: 16 litres):

80 mL Phosphate buffer (final conc.: 5 mM),
16 mL NaCl solution (final conc.: 5 mM),
20 mL $NH_4Cl$ solution (final conc.: 5 mM),
2 mL, 6.5 mL or 8 mL Haloperoxidase solution (final conc.: 8 mg/L, 26 mg/L or 32 mg/L),
9.8 mL or 13.06 mL Hydrogen peroxide (final conc.: 6 mM or 8 mM), and 1.23 mL commercial non-ionic surfactant.

The 5 independent examples shown in Tables 1 to 5 were carried out using duplicates or triplicates of the polyester sutures and porcelain cylinders. The polyester sutures and porcelain cylinders were placed in an aluminium basket, which was placed on a shelf in the Washer Disinfector.

After the "End"-step, the polyester sutures and porcelain cylinders were carefully transferred to a tube containing 1% Tween 80 and glass beads, and the tubes were shaken for 15 minutes at 300 rpm, to recover the remaining spores.

Subsequently, a dilution series was made in MilliQ water, and 100 $\mu$L from the dilutions $10^0$ to $10^{-3}$ were plated on LB agar plates. The plates were incubated for 48 hours at 37°C, and the average number of colony forming units (CFU) on each plate was registered.

The number of colonies was used to calculate the log reduction (kill) in the number of recoverable bacteria from the polyester sutures and porcelain cylinders. The results are listed in the tables below. The conditions used in each experiment are indicated above each of the tables.

Table 1 (washer disinfector tests). Number of CFU on plates. Treatment: 60°C for 6 hours.

| 32 mg/L haloperoxidase, 8 mM $H_2O_2$ | | | | | |
|---|---|---|---|---|---|
| **Sample** | **Dilution** | | | | **Total** |
| | No dilution ($10^0$) | $10^{-1}$ | $10^{-2}$ | $10^{-3}$ | |
| polyester suture 1 | 0 | 0 | 0 | 0 | 0 |
| polyester suture 2 | 0 | 0 | 0 | 0 | 0 |
| polyester suture 3 | 0 | 0 | 0 | 0 | 0 |
| porcelain cylinder 1 | 0 | 0 | 0 | 0 | 0 |
| porcelain cylinder 2 | 0 | 0 | 0 | 0 | 0 |
| porcelain cylinder 3 | 0 | 0 | 0 | 0 | 0 |

Table 2 (washer disinf. tests). Number of CFU on plates. Treatment: 60°C for 2 hours.

| 26 mg/L haloperoxidase, 6 mM $H_2O_2$ | | | | | |
|---|---|---|---|---|---|
| **Sample** | **Dilution** | | | | **Total** |
| | No dilution ($10^0$) | $10^{-1}$ | $10^{-2}$ | $10^{-3}$ | |
| polyester suture 1 | 0 | 0 | 0 | 0 | 0 |
| polyester suture 2 | 0 | 0 | 0 | 0 | 0 |
| polyester suture 3 | 0 | 0 | 0 | 0 | 0 |
| porcelain cylinder 1 | 0 | 0 | 0 | 0 | 0 |
| porcelain cylinder 2 | 0 | 0 | 0 | 0 | 0 |
| porcelain cylinder 3 | 0 | 0 | 0 | 0 | 0 |

Table 3 (washer disinf. tests). Number of CFU on plates. Treatment: 50°C for 2 hours.

| 32 mq/L haloperoxidase, 8 mM $H_2O_2$ | | | | | |
|---|---|---|---|---|---|
| Sample | Dilution | | | | Total |
| | No dilution ($10^0$) | $10^{-1}$ | $10^{-2}$ | $10^{-3}$ | |
| polyester suture 1 | 0 | 0 | 0 | 0 | 0 |
| polyester suture 2 | 0 | 0 | 0 | 0 | 0 |
| polyester suture 3 | 0 | 0 | 0 | 0 | 0 |
| porcelain cylinder 1 | 0 | 0 | 0 | 0 | 0 |
| porcelain cylinder 2 | 0 | 0 | 0 | 0 | 0 |
| porcelain cylinder 3 | 0 | 0 | 0 | 0 | 0 |

Table 4 (washer disinf. tests). Number of CFU on each plate. Treatment: 40°C for 18 hours.

| 8 mg/L haloperoxidase, 8 mM $H_2O_2$ | | | | | |
|---|---|---|---|---|---|
| Sample | Dilution | | | | Total |
| | No dilution ($10^0$) | $10^{-1}$ | $10^{-2}$ | $10^{-3}$ | |
| polyester suture 1 | 0 | 0 | 0 | 0 | 0 |
| polyester suture 2 | 0 | 0 | 0 | 0 | 0 |
| polyester suture 3 | 0 | 0 | 0 | 0 | 0 |
| porcelain cylinder 1 | 0 | 0 | 0 | 0 | 0 |
| porcelain cylinder 2 | 0 | 0 | 0 | 0 | 0 |
| porcelain cylinder 3 | 0 | 0 | 0 | 0 | 0 |

The results shown in Tables 1 to 4 demonstrate that the haloperoxidase system of the invention has a clear and significant sporicidal effect. No CFU were counted on any of the dilutions, thus minimum a log 6 reduction was obtained by treating the spore carriers (polyester sutures and porcelain cylinders) with the haloperoxidase system.

Table 5 (washer disinf. tests). Number of CFU on plates. Treatment: 40°C for 4 hours.

| 32 mg/L haloperoxidase, 8 mM $H_2O_2$ | | | | | |
|---|---|---|---|---|---|
| Sample | Dilution | | | | Total |
| | No dilution ($10^0$) | $10^{-1}$ | $10^{-2}$ | $10^{-3}$ | |
| polyester suture 1 | 1 | 0 | 0 | 0 | 50 |
| polyester suture 2 | 0 | 0 | 0 | 0 | 0 |
| porcelain cylinder 1 | 0 | 0 | 0 | 0 | 0 |
| porcelain cylinder 2 | 0 | 0 | 0 | 0 | 0 |

The results shown in Table 5 also demonstrate that the haloperoxidase system of the invention has a clear and significant sporicidal effect. 50 CFU were registered from one of the porcelain cylinders, which shows a log 5 reduction of spores after treatment with the haloperoxidase system. The other spore carriers showed at least a log 6 reduction in the number of spores.

All of the above examples clearly demonstrate that by using the haloperoxidase system in a Washer Disinfector for high level disinfection, a significant reduction in the number of spores can be obtained.

**EXAMPLE 17**

Inactivation of poliovirus

**Virus and cells**

The poliovirus strain Sabin (Type 2H.010704) obtained from WHO was propagated in VERO cells at 34.5°C, 5% $CO_2$ using Eagles MEM 2% FCS, 100 IU/mL penicillin, 100 mg/mL streptomycin, and 20 $\mu$g/mL gentamycin. Culture supernatant was filtered (0.45 nm), aliquoted and stored at -80°C until use.

**Stock solutions**

The following stock solutions were prepared.

Solution 1: 22.8 mM NaCl and 7.2 mM $NH_4Cl$ in 20 mM DMG (Sigma D4379) buffer pH 7.0;
Solution 2: 10% $H_2O_2$ in MilliQ water;
Solution 3: 8000 ppm haloperoxidase from *Curvularia verrucolosa* (see SEQ ID NO:2 in WO 97/04102) in 20 mM DMG buffer pH 7.0;

Phosphate buffered saline

**Disinfectant solution**

To create approximately 50 mL of active disinfecting solution, the following was mixed:

50 mL solution 1; 62 $\mu$L solution 2; and 100 $\mu$L solution 3.

The undiluted disinfecting solution was further diluted with phosphate buffered saline (PBS) to give 1:10 and 1:100 solutions.

**Cell culture media**

Eagles MEM media supplemented with 4% fetal calf serum, 100 IU/mL penicillin, 100 mg/mL streptomycin and 20 $\mu$g/mL gentamycin.

**Treatment of virus**

In a microtiter plate 50 $\mu$L of the test solution was mixed with 50 $\mu$L of a diluted polio virus stock in PBS and incubated at 34.5°C for 1 hour.
In order to compare the enzymatic disinfection solution to a known disinfectant, sodium hypochlorite was used as a control at concentrations of 0.5%, 0.05% and 0.005%
For all treatments a control without virus addition was made in order to identify any cytotoxic effects of the test solutions on the cell line. In addition a positive control of virus infectivity was included in which no disinfecting solution was added.
To assay for remaining infective virus after the treatment, 100 $\mu$L of the test solutions and 100 $\mu$L Eagles minimal media with 4% FCS was added to a fresh microtiter plate in which confluent growth of the RD cell line (human rhabdomyosarcoma) had been established. This plate was incubated for 5 days at 34.5°C and 5% $CO_2$, before infectivity and cytotoxicity was scored by an expert evaluator using a microscope.

Table 1 (virus tests). Results from antiviral testing of Poliovirus Sabin (Type 2H.010704).

|  |  | 1 | 2 | 3 | 4 | 5 | 6 | 7 |
|---|---|---|---|---|---|---|---|---|
|  |  | Control (no enzyme) | Enzyme 1:1 | Enzyme 1:10 | Enzyme 1:100 | Hypo-chlorite 0.5% | Hypo-chlorite 0.05% | Hypo-chlorite 0.005% |
| No virus added |  | N | N* | N | N | N* | N | N |
|  |  | N | N* | N | N | N* | N | N |
|  |  | N | N* | N | N | N* | N | N |
|  |  | N | N* | N | N | N* | N | N |
| Virus added |  | I | N* | N | N | N* | N | N |
|  |  | I | N* | N | N | N* | N | N |
|  |  | I | N* | N | N | N* | N | N |
|  |  | I | N* | N | N | N* | N | N |

Enzyme: Haloperoxidase

Hypochlorite:     Sodium hypochlorite
I:                Infection
N:                No infection
N*:               No infection with a slight toxic effect
T:                Toxic

The results show that the method of the invention inhibited poliovirus (lane 2-4) to the same extend as sodium hypochlorite (lane 5-7). The assay was performed in duplicate. The enzyme system showed strong virucidal activity, even down to a 1:100 dilution.

**EXAMPLE 18**

Inactivation of HIV-1 virus

**Virus and cells**

The HIV-1 strain HTLV-IIIB (NIH AIDS Research and Reference Program) was propagated in H9 cells (NIH AIDS Research and Reference Program) at 37°C, 5% $CO_2$ using RPMI 1640 with 10% heat inactivated fetal calf serum (FCS), 100 IU/mL penicillin, 100 mg/mL streptomycin, 20 μg/mL gentamycin, and 10 IU/mL nystatin. Culture supernatant was filtered (0.45 nm), aliquoted and stored at -80°C until use.

**Stock solutions**

The following stock solutions were prepared.

Solution 1: 22.8 mM NaCl and 7.2 mM $NH_4Cl$ in 20 mM DMG (Sigma D4379) buffer pH 7.0;
Solution 2: 10% $H_2O_2$ in MilliQ water;
Solution 3: 8000 ppm haloperoxidase from *Curvularia verrucolosa* (see SEQ ID NO:2 in WO 97/04102) in 20 mM DMG buffer pH 7.0;

Phosphate buffered saline

**Disinfectant solution**

To create approximately 50 mL of active disinfecting solution, the following was mixed:

50 mL solution 1; 62 μL solution 2; and 100 μL solution 3.

The undiluted disinfecting solution was further diluted with phosphate buffered saline (PBS) to give 1:10 and 1:100 solutions.

**Cell culture media**

RPMI1640 Glutamax (Sigma R8758) supplemented with 100 IU/mL penicillin, 100 mg/mL Streptomycin, 20 μg/mL gentamycin, 10 IU/mL Nystatin and 5% fetal calf serum.

**MTT reagent**

50 mg MTT (Sigma M5655)
10 mL PBS pH 7.4

**MTT stop reagent**

10 mL Triton X-100 (Sigma T8787)
4 mL 1M HCl
isopropanol to 100 mL

**Treatment of virus**

In a microtiter plate 50 μL of the test solution was mixed with 50 μL of a diluted HIV virus stock in PBS and incubated at 37°C for 5 min.

In order to compare the enzymatic disinfection solution to a known disinfectant, sodium hypochlorite was used as a control at concentrations of 0.5%, 0.05% and 0.005%.

For all treatments a control without virus addition was made in order to identify any cytotoxic effects of the test solutions on the cell line. In addition a positive control of virus infectivity was included in which no disinfecting solution was added.

To assay for remaining infective virus after the treatment, 100 μL of the test solutions and 100 μL RPMI1640 cell media with 5% FCS containing $0.3 \times 10^3$ MT4 cells/mL was added to a fresh microtiter plate. This plate was incubated for 6 days at 37°C and 5% $CO_2$, before viability was measured in a MTT assay. 30 μL MTT reagent was added to each well and the plate was incubated for 1 hour at 37°C. 150 μL of the solution was removed and replaced with 130 μL MTT stop solution and the content was mixed.

Absorbance was measured in a plate reader at 540 nm with absorbance at 690 nm as reference. Based on the absorbance, each well was scored to be either infected or non-infected in the virus containing wells; and toxic or non-infected in the control wells for cytotoxicity.

Table 2 (virus tests). Results from antiviral testing of HIV-1 virus (Strain HTLV-IIIB).

| | 1 | 2 | 3 | 4 | 5 | 6 | 7 |
|---|---|---|---|---|---|---|---|
| | Control (no enzyme) | Enzyme 1:1 | Enzyme 1:10 | Enzyme 1:100 | Hypo-chlorite 0.5% | Hypo-chlorite 0.05% | Hypo-chlorite 0.005% |
| No virus added | N | T | T | N | T | T | T |
| | N | T | T | N | T | T | T |
| | N | T | T | N | T | T | T |
| | N | T | T | N | T | T | T |

(continued)

| | 1 | 2 | 3 | 4 | 5 | 6 | 7 |
|---|---|---|---|---|---|---|---|
| | Control (no enzyme) | Enzyme 1:1 | Enzyme 1:10 | Enzyme 1:100 | Hypo-chlorite 0.5% | Hypo-chlorite 0.05% | Hypo-chlorite 0.005% |
| Virus added | I | T | T | N | T | T | T |
| | I | T | T | N | T | T | T |
| | I | T | T | N | T | T | T |
| | I | T | T | N | T | T | T |

Enzyme:           Haloperoxidase
Hypochlorite:      Sodium hypochlorite
I:               Infection
N:              No infection
N*:            No infection with a slight toxic effect
T:              Toxic

The results show that the method of the invention had an antiviral effect on HIV virus in a 1:100 dilution (lane 4) during an incubation period of 5 min.

Reference: (1983) Rapid colorimetric assay for cellular growth and survival: Application to profileration and cytotoxicity assays. J. Imm. Meth, 65, 55-63.

## EXAMPLE 19

Killing of *Mycobacterium bovis* with haloperoxidase from *Curvularia verrucolosa*

The objective of this assay was to evaluate the tuberculocidal effectiveness of a product against *Mycobacterium bovis* - BCG following the AOAC Tuberculocidal Activity.

## MATERIALS

Test organism: *Mycobacterium bovis* - BCG; obtained from Organon Teknika, Durham, USA.
Growth medium: Modified Proskauer-Beck Medium (MPB)

## Recovery Media

Neutralizer:           Letheen Broth + 1.0% Sodium Thiosulfate + 0.01 % Catalase
Subculture Medium:   Modified Proskauer-Beck Medium (MPB)
                       Middlebrook 7H9 Broth (7H9)
Agar Plates:           Middlebrook 7H11 Agar

## Carriers

Porcelain penicylinders (O.D. 8 mm ±1, I.D. 6 mm ±1, length 10 mm ±1) were washed with 1-5% Triton X-100 and rinsed with water at least four times, until no soap residue was present. Following washing, the carriers were macroscopically inspected for chip and cracks. Carriers with visible chips or cracks were discarded. Carriers were placed in a vessel and sterilized for 2 hours in a 180°C air oven.

## TEST METHOD

## Preparation of Test Substance

A total volume of 10.0 mL test substance was prepared in each 25 x 150 mm Morton Closure tube by mixing:

9.9 mL of solution A (125 $\mu$L of 400 mM NaCl, 250 $\mu$L of 200 mM NH$_4$Cl, 50 $\mu$L of 1000 mM phosphate buffer, 0.77 mg of BASF LF 900, 4475$\mu$ L MilliQ water and 5000 $\mu$L 40°dH H$_2$O) resulting in a final concentration of 5 mM NaCl, 5 mM NH$_4$Cl, 5 mM phosphate buffer and 0.077 g/L BASF LF 900;

0.05 mL of solution B (50 $\mu$L of 1600 ppm haloperoxidase from *Curvularia verrucolosa* (see SEQ ID NO:2 in WO 97/04102)) resulting in a final concentration of 8 ppm enzyme; and

0.05 mL of solution C (50 $\mu$L of 1600 mM H$_2$O$_2$) resulting in a final concentration of 8 mM H$_2$O$_2$.

The tubes were then placed in a 40.0°C water bath to equilibrate for 22 minutes. Testing was performed in duplicate. The test substance was homogenous as determined by visual observation and was used within one hour of preparation.

**Preparation of Test Organism**

A stock culture of the test organism, *Mycobacterium bovis* - BCG, was maintained on 7H11 agar medium. From the stock culture, the organism was transferred into Modified Proskauer-Beck broth and incubated for 22 days at 35-37°C. Following incubation, a 1.00 mL aliquot of 0.1% Polysorbate 80 in saline was added to the suspension. The culture broth was transferred to a sterile tissue grinder and thoroughly ground. This suspension was diluted in 10.0 mL of Modified Proskauer-Beck growth media prior to carrier contamination. The final percent transmittance of the suspension was determined to be at 9.78%T using a spectrophotometer calibrated to 650 nm.

**Contamination of Carriers**

The penicylinders were immersed for 15 minutes in the ground culture at a ratio of 1 carrier per 1.0 mL culture. The carriers were then dried on filter paper in a sterile Petri dish at 35-37°C for 30 minutes at 40% relative humidity. The drying conditions (temperature and humidity) were appropriate for the test organism for the purpose of obtaining maximum survival following drying.

**Exposure Conditions**

Ten carriers per replicate of test substance were tested. Each contaminated and dried carrier was placed into a separate tube containing 10.0 mL of the test substance at its use-dilution for the 15 minute exposure time at 40.0°C.

**Test System Recovery**

Each medicated carrier was transferred by wire hook at staggered intervals to 10 mL of neutralizer. The neutralized carrier was then transferred to a tube containing 20 mL Modified Proskauer-Beck subculture medium. Furthermore, a 2.0 mL aliquot of the neutralizing subculture medium was individually subcultured using 20 mL of Middlebrook 7H9 broth.

**Incubation**

All broth subcultures were incubated at 35-37°C under aerobic conditions. The subculture plates were placed in plastic bags and incubated for 15 days at 35-37°C prior to examination. The broth subculture tubes were visually examined for growth following a 30, 60 and 90 day incubation period.

Representative subcultures demonstrating growth ($\geq$20%) were stained using an AFB fluorescent stain to confirm identity of test organism.

**STUDY CONTROLS**

**Purity Control**

A "streak plate for isolation" was performed on the organism culture, and following incubation, examined in order to confirm the presence of a pure culture. The acceptance criterion for this study control is a pure culture demonstrating colony morphology typical of the test organism.

**Carrier Sterility Control**

A representative uninoculated carrier was added to the neutralizer. The carrier was transferred from the neutralizer to Modified Proskauer-Beck. Aliquots (2.0 mL) of the neutralizer were individually subcultured into 20 mL of Middlebrook 7H9 broth in a manner consistent with the test procedure. The subculture broths were incubated and examined for growth. The acceptance criterion for this study control is lack of growth.

**Subculture Medium Sterility Control**

A representative sample of Modified Proskauer-Beck and Middlebrook 7H9 broth were incubated and visually examined. The acceptance criterion is lack of growth.

**Neutralizer Sterility Control**

Aliquots (2.0 mL) of the neutralizer were added to an uninoculated representative sample of each subculture medium, Modified Proskauer-Beck and Middlebrook 7H9 broth, incubated and visually examined. The acceptance criterion is lack of growth.

**Viability Control**

A representative inoculated carrier was added to the neutralizer. The carrier was then transferred to Modified Proskauer-Beck. Aliquots (2.0 mL) of the neutralizer were then subcultured to Middlebrook 7H9 broth in a manner consistent with the test procedure. The subculture broths were incubated and examined for growth. The acceptance criterion for this study control is growth.

**Neutralization Confirmation Control**

The neutralization of the test substance was confirmed by exposing sterile carriers (representing not less than 10% of the total number of test carriers) to the test substance and transferring them to primary subcultures containing 10 mL of neutralizer. The carriers were subcultured to Modified Proskauer-Beck, identically to the test procedure. Aliquots (2.0 mL) of the neutralizer were then subcultured to a corresponding number of Middlebrook 7H9 broth in a manner consistent with the test procedure. The subcultures containing the exposed carriers and those to which the 2.0 mL aliquots of neutralizer had been subcultured were inoculated with low levels of test organism, incubated under test conditions and visually examined for the presence of growth. This control was performed with multiple replicates using different dilutions of the test organism. A standardized spread plate procedure was run concurrently in order to enumerate the number of CFU actually added. The control result is reported using data from the most appropriate dilution.
The acceptance criterion for this study control is growth following inoculation with low levels of test organism ($\leq$1000 CFU) in at least one of the three types of subculture media.

**Carrier Population Control**

Contaminated carriers were transferred to a sterile container of Modified Proskauer-Beck at a ratio of one carrier to 10 mL of medium and vortex mixed. This suspension was serially diluted and plated in duplicate on Middlebrook 7H11 agar plates using standard microbiological techniques. Following incubation, the organism plates were observed to enumerate the concentration of the test organism present at the time of testing. The acceptance criterion for this study control is a minimum of $1.0 \times 10^{-4}$ CFU/carrier.

**Calculations**

Carrier Population Control Calculation:

$$\text{CFU/carrier} = \frac{(\text{average number of colonies/plate@dilution}) \times (\text{dilution factor}) \times (\text{volume MPB})}{(\text{number of carriers tested}) \times (\text{volume plated})}$$

The carrier population was calculated and reported using data from the most appropriate dilution(s).

## CONDITIONS

Exposure time: 15 minutes
Exposure temperature: 40±2°C (40.0°C)
Haloperoxidase concentration: 8 ppm (8 mg enzyme protein/L) and 5 mM NaCl, 5 mM NH$_4$Cl, 5 mM phosphate buffer, 0.077 g/L surfactant LF900, and 8 mM H$_2$O$_2$.
Test organism: *Mycobacterium bovis* - BCG; 1.12 x 10$^5$ CFU/carrier (enumerated on day 15)

## RESULTS

Viability of the test organism and sterility of the media were confirmed before carrying out the experiments (as explained above).

Table 1 (*Mycobacterium* tests). Replicate 1.

| Subculture Media | Volume subcultured | Total number of carriers | Number of carriers showing growth of *Mycobacterium bovis* | |
|---|---|---|---|---|
| | | | 30 days | 62 days |
| Modified Proskauer-Beck | NA | 10 | 0 | 2 |
| Middlebrook 7H9 Broth | 2.0 mL | 10 | 0 | 0 |

Growth of the test organism was qualitatively observed after 30 and 62 days of incubation. After 30 days of incubation no growth was observed on any of the carriers or in the subcultured media. After 62 days of incubation 2/10 (two out of ten) carriers in modified Proskauer-Beck medium showed growth, which was validated to be the test organism *Mycobacterium bovis* - BCG. In the subcultured medium (Middlebrook 7H9 broth) none (0/10) of the tubes showed any growth. The haloperoxidase treatment has significantly delayed the growth of the test organism, and in 2/10 (80%) of the carriers no growth was observed.

Table 2 (*Mycobacterium* tests). Replicate 2.

| Subculture Media | Volume subcultured | Total number of carriers | Number of carriers showing growth of *Mycobacterium bovis* | | |
|---|---|---|---|---|---|
| | | | 30 days | 62 days | 90 days |
| Modified Proskauer-Beck | NA | 10 | 0 | 0 | 0 |
| Middlebrook 7H9 Broth | 2.0 mL | 10 | 0 | 0 | 1 |

In replicate 2, the carriers were also observed for growth after 30, 52 and 90 days respectively. After 30 and 63 days of incubation none of the carriers or subcultured carriers showed any growth. After 90 days of incubation none of the carriers (0/10) showed any growth, whereas one (1/10) of the subcultured carriers showed growth, which was identified to be the *Mycobacterium bovis* - BCG.

The haloperoxidase system has in short time (15 min) at medium temperature (40°C) at low dosage (8ppm) significantly showed kill efficacy towards the test organism *Mycobacterium bovis* - BCG, as replicate 1 gave a kill efficacy of 80% and replicate 2 showed 100% kill efficacy of the test organism attached to the porcelain penicylinder carriers.

## Claims

**1.** A method of disinfecting or sterilizing a device in a washer disinfector, comprising supplying a haloperoxidase, hydrogen peroxide, chloride and/or bromide ions, and ammonium ions to the washer-disinfector.

**2.** A method according to claim 1, comprising supplying said haloperoxidase, hydrogen peroxide, chloride and/or bromide ions, and ammonium ions to a washing chamber (2) of said washer disinfector (1) during an enzymatic disinfection phase, said enzymatic disinfection phase comprising contacting said haloperoxidase, hydrogen peroxide, chloride and/or bromide ions, and ammonium ions with said device (D) in said washing chamber (2) at a temperature which is 65 degrees Celsius or lower.

**3.** A method according to claim 2, wherein at least a portion of said enzymatic disinfection phase occurs at a temperature which is in the range of 20 to 65 degrees Celsius.

**4.** A method according to any one of claims 2-3, wherein said enzymatic disinfection phase occurs for a period of time which is 5-90 minutes, said period of time starting at the moment of introducing the haloperoxidase, hydrogen peroxide, chloride and/or bromide ions, and ammonium ions into the washing chamber (2), and ending the moment of draining the haloperoxidase, hydrogen peroxide, chloride and/or bromide ions, and ammonium ions from the washing chamber (2).

**5.** A method according to any one of claims 2-4, wherein said enzymatic disinfection phase is preceded by a washing phase, during which a detergent and water is added to said washing chamber (2) for treating said device (D) at a temperature in the range of 20 to 85 degrees Celsius.

**6.** A method according to any one of claims 2-5, wherein said enzymatic disinfection phase is followed by a post enzymatic disinfection rinse phase, in which a cleaned water, having passed through a filter having a pore size of maximum 0.2 micrometer, or having at least the corresponding degree of purity, is supplied to said washing chamber (2) to rinse said device (D).

**7.** A method according to any one of claims 2-6, wherein a drying phase is carried out after said enzymatic disinfection phase and before the device is removed from the washing chamber (2), said drying phase comprising blowing air having a temperature of 90 degrees Celsius or lower into the washing chamber (2).

**8.** A method according to any one of the preceding claims, wherein the temperature inside a washing chamber (2) of the washer disinfector (1) is 65 degrees Celsius or lower during the entire process of sterilizing or disinfecting said device (D).

**9.** A method according to any one of the preceding claims, said method comprising at least the following consecutive phases;
at least one washing phase for treating said device (D) with a detergent,
at least one post wash rinse phase for rinsing said device (D) with water,
at least one enzymatic disinfection phase for treating said device (D) with said haloperoxidase, hydrogen peroxide, chloride and/or bromide ions, and ammonium ions, and
at least one post enzymatic disinfection rinse phase for rinsing said device (D) with water.

**10.** Washer disinfector for disinfecting or sterilizing a device, **characterised in** said washer disinfector (1) comprising a washing chamber (2) which is adapted for housing said device (D), and
an arrangement (34, 36, 38, 40, 42, 44) containing a haloperoxidase, hydrogen peroxide, chloride and/or bromide ions, and ammonium ions, and arranged for supplying those substances to said washing chamber (2).

**11.** Washer disinfector according to claim 10, said washer disinfector (1) comprising a haloperoxidase supply arrangement (34, 36, 38) which is operative for storing and supplying a haloperoxidase solution containing haloperoxidase, and a hydrogen peroxide supply arrangement (40, 42, 44) which is operative for storing and supplying a hydrogen peroxide solution containing hydrogen peroxide and/or a hydrogen peroxide precursor.

**12.** Washer disinfector according to any one of claims 10-11, said washer disinfector (1) comprising a control unit (62) which is operative for controlling the temperature inside said washing chamber (2) to be 65 degrees Celsius or lower during the entire process of disinfecting or sterilizing said device (D).

**13.** Washer disinfector according to any one of claims 10-12, said washer disinfector (1) comprising a water supply arrangement (8, 10, 12), which is operative for supplying a cleaned water, having passed through a filter having a pore size of maximum 0.2 micrometer, or having at least the corresponding degree of purity, to said washing chamber (2) for rinsing said device (D) after having been treated with said haloperoxidase, hydrogen peroxide, chloride and/or bromide ions, and ammonium ions.

14. Washer disinfector according to any one of claims 10-13, said washer disinfector (1) comprising a detergent supply arrangement (28, 30, 32) which is operative for supplying a detergent to said washing chamber (2) prior to supplying said haloperoxidase, hydrogen peroxide, chloride and/or bromide ions, and ammonium ions to said washing chamber (2).

15. Use of a haloperoxidase, hydrogen peroxide, chloride and/or bromide ions, and ammonium ions in a washer disinfector (1) for disinfecting or sterilizing a device (D).

16. The use according to claim 15, for disinfecting a medical device (D).

17. The use according to any one of claims 15 and 16, wherein the temperature inside a washing chamber (2) of the washer disinfector (1) is 65 degrees Celsius or lower during the disinfection of the device (D).


**Patentansprüche**

1. Verfahren zum Desinfizieren oder Sterilisieren einer Vorrichtung in einem Wasch-Desinfektionsgerät, umfassend ein Zuleiten von Haloperoxidase, Wasserstoffperoxid, Chlorid- und/oder Bromidionen und Ammoniumionen zum Wasch-Desinfektionsgerät.

2. Verfahren nach Anspruch 1, umfassend ein Zuleiten der Haloperoxidase, des Wasserstoffperoxids, der Chlorid- und/oder Bromidionen und Ammoniumionen zu einer Waschkammer (2) des Wasch-Desinfektionsgeräts (1) während einer enzymatischen Desinfektionsphase, wobei die enzymatische Desinfektionsphase ein Kontaktieren der Haloperoxidase, des Wasserstoffperoxids, der Chlorid- und/oder Bromidionen und Ammoniumionen mit der Vorrichtung (D) in der Waschkammer (2) bei einer Temperatur umfasst, die 65 Grad Celsius oder weniger ist.

3. Verfahren nach Anspruch 2, wobei zumindest ein Teil der enzymatischen Desinfektionsphase bei einer Temperatur erfolgt, die im Bereich von 20 bis 65 Grad Celsius liegt.

4. Verfahren nach einem der Ansprüche 2-3, wobei die enzymatische Desinfektionsphase über einen Zeitraum erfolgt, der 5 bis 90 Minuten ist, wobei der Zeitraum in dem Moment der Einleitung der Haloperoxidase, des Wasserstoffperoxids, der Chlorid- und/oder Bromidionen und Ammoniumionen in die Waschkammer (2) beginnt und in dem Moment des Ablassens der Haloperoxidase, des Wasserstoffperoxids, der Chlorid- und/oder Bromidionen und Ammoniumionen aus der Waschkammer (2) endet.

5. Verfahren nach einem der Ansprüche 2-4, wobei der enzymatischen Desinfektionsphase eine Waschphase vorangeht, in der ein Reinigungsmittel und Wasser der Waschkammer (2) beigegeben werden, um die Vorrichtung (D) bei einer Temperatur im Bereich von 20 bis 85 Grad Celsius zu behandeln.

6. Verfahren nach einem der Ansprüche 2-5, wobei auf die enzymatische Desinfektionsphase eine Spülphase nach enzymatischer Desinfektion folgt, in der ein gereinigtes Wasser, das durch ein Filter mit einer Porengröße von maximal 0,2 Mikrometer gegangen ist oder zumindest den entsprechenden Reinheitsgrad aufweist, der Waschkammer (2) zum Spülen der Vorrichtung (D) zugeleitet wird.

7. Verfahren nach einem der Ansprüche 2-6, wobei ein Trocknungsschritt nach der enzymatischen Desinfektionsphase und bevor die Vorrichtung der Waschkammer (2) entnommen wird ausgeführt wird, wobei die Trocknungsphase ein Einblasen von Luft mit einer Temperatur von 90 Grad Celsius oder weniger in die Waschkammer (2) umfasst.

8. Verfahren nach einem der vorangehenden Ansprüche, wobei die Temperatur im Inneren einer Waschkammer (2) des Wasch-Desinfektionsgeräts (1) während des gesamten Prozesses der Sterilisierung und Desinfizierung der Vorrichtung (D) 65 Grad Celsius oder weniger ist.

9. Verfahren nach einem der vorangehenden Ansprüche, wobei das Verfahren zumindest die folgenden, aufeinander folgenden Phasen umfasst:

zumindest eine Waschphase zum Behandeln der Vorrichtung (D) mit einem Reinigungsmittel,

zumindest eine Spülphase nach dem Waschen zum Spülen der Vorrichtung (D) mit Wasser,
zumindest eine enzymatische Desinfektionsphase zum Behandeln der Vorrichtung (D) mit der Haloperoxidase,
dem Wasserstoffperoxid, den Chlorid- und/oder Bromidionen und Ammoniumionen und
zumindest eine Spülphase nach enzymatischer Desinfektion zum Spülen der Vorrichtung (D) mit Wasser.

10. Wasch-Desinfektionsgerät zum Desinfizieren oder Sterilisieren einer Vorrichtung, **dadurch gekennzeichnet, dass** das Wasch-Desinfektionsgerät (1) umfasst
eine Waschkammer (2), die zur Aufnahme der Vorrichtung (D) ausgebildet ist, und
eine Anordnung (34, 36, 38, 40, 42, 44), die eine Haloperoxidase, Wasserstoffperoxid, Chlorid- und/oder Bromidionen und Ammoniumionen enthält und zum Zuleiten dieser Substanzen zur Waschkammer (2) angeordnet ist.

11. Wasch-Desinfektionsgerät nach Anspruch 10, wobei das Wasch-Desinfektionsgerät (1) eine Haloperoxidase-Zuleitungsanordnung (34, 36, 38), die zum Lagern und Zuleiten einer Haloperoxidaselösung, die Haloperoxidase enthält, betriebsbereit ist, und eine Wasserstoffperoxid-Zuleitungsanordnung (40, 42, 44), die zum Lagern und Zuleiten einer Wasserstoffperoxidlösung, die Wasserstoffperoxid und/oder einen Wasserstoffperoxidvorläufer enthält, betriebsbereit ist, umfasst.

12. Wasch-Desinfektionsgerät nach einem der Ansprüche 10-11, wobei das Wasch-Desinfektionsgerät (1) eine Steuereinheit (62) umfasst, die zum Steuern der Temperatur im Inneren der Waschkammer (2) auf 65 Grad Celsius oder weniger während des gesamten Verfahrens zum Desinfizieren oder Sterilisieren der Vorrichtung (D) betriebsbereit ist.

13. Wasch-Desinfektionsgerät nach einem der Ansprüche 10-12, wobei das Wasch-Desinfektionsgerät (1) eine Wasserzuleitungsanordnung (8, 10, 12) umfasst, die betriebsbereit ist zum Zuleiten von gereinigtem Wasser, das durch ein Filter mit einer Porengröße von maximal 0,2 Mikrometer gegangen ist oder zumindest den entsprechenden Reinheitsgrad aufweist, zur Waschkammer (2) zum Spülen der Vorrichtung (D), nachdem sie mit der Haloperoxidase, dem Wasserstoffperoxid, den Chlorid- und/oder Bromidionen und Ammoniumionen behandelt wurde.

14. Wasch-Desinfektionsgerät nach einem der Ansprüche 10-13, wobei das Wasch-Desinfektionsgerät (1) eine Reinigungsmittelzuleitungsanordnung (28, 30, 32) umfasst, die zum Zuleiten eines Reinigungsmittels zur Waschkammer (2) vor dem Zuleiten der Haloperoxidase, des Wasserstoffperoxids, der Chlorid- und/oder Bromidionen und Ammoniumionen zur Waschkammer (2) betriebsbereit ist.

15. Verwendung von Haloperoxidase, Wasserstoffperoxid, Chlorid- und/oder Bromidionen und Ammoniumionen in einem Wasch-Desinfektionsgerät (1) zum Desinfizieren oder Sterilisieren einer Vorrichtung (D).

16. Verwendung nach Anspruch 15 zum Desinifizieren einer medizinischen Vorrichtung (D).

17. Verwendung nach einem der Ansprüche 15 und 16, wobei die Temperatur im Inneren einer Waschkammer (2) des Wasch-Desinfektionsgeräts (1) während der Desinfektion der Vorrichtung (D) 65 Grad Celsius oder weniger ist.

## Revendications

1. Procédé de désinfection ou de stérilisation d'un dispositif dans un laveur désinfecteur, comprenant de fournir une haloperoxydase, du peroxyde d'hydrogène, des ions chlorure et/ou bromure, et des ions ammonium au laveur désinfecteur.

2. Procédé selon la revendication 1, comprenant de fournir ladite haloperoxydase, ledit peroxyde d'hydrogène, lesdits ions chlorure et/ou bromure, et lesdits ions ammonium à une chambre de lavage (2) dudit laveur désinfecteur (1) pendant une phase de désinfection enzymatique, ladite phase de désinfection enzymatique comprenant de mettre en contact ladite haloperoxydase, ledit peroxyde d'hydrogène, lesdits ions chlorure et/ou bromure, et lesdits ions ammonium avec ledit dispositif (D) dans ladite chambre de lavage (2) à une température qui est de 65 degrés Celsius ou inférieure.

3. Procédé selon la revendication 2, dans lequel au moins une portion de ladite phase de désinfection enzymatique se produit à une température qui est dans la plage de 20 à 65 degrés Celsius.

**4.** Procédé selon l'une quelconque des revendications 2 - 3, dans lequel ladite phase de désinfection enzymatique se produit pendant une période de temps qui est de 5 - 90 minutes, ladite période de temps commençant au moment de l'introduction de l'haloperoxydase, du peroxyde d'hydrogène, des ions chlorure et/ou bromure, et des ions ammonium dans la chambre de lavage (2), et finissant au moment de la vidange de l'haloperoxydase, du peroxyde d'hydrogène, des ions chlorure et/ou bromure, et des ions ammonium de la chambre de lavage (2).

**5.** Procédé selon l'une quelconque des revendications 2 - 4, dans lequel ladite phase de désinfection enzymatique est précédée par une phase de lavage, pendant laquelle un détergeant et de l'eau sont ajoutés à ladite chambre de lavage (2) pour traiter ledit dispositif (D) à une température dans la plage de 20 à 85 degrés Celsius.

**6.** Procédé selon l'une quelconque des revendications 2 - 5, dans lequel ladite phase de désinfection enzymatique est suivie par une phase de rinçage ultérieure à la désinfection enzymatique, dans laquelle une eau propre, ayant passée au travers d'un filtre ayant une taille de pore de 0,2 micromètre au maximum, ou ayant au moins le degré correspondant de pureté, est fournie à ladite chambre de lavage (2) pour rincer ledit dispositif (D).

**7.** Procédé selon l'une quelconque des revendications 2 - 6, dans lequel une phase de séchage est effectuée après ladite phase de désinfection enzymatique et avant que le dispositif soit retiré de la chambre de lavage (2), ladite phase de séchage comprenant de souffler de l'air ayant une température de 90 degrés Celsius ou inférieure dans la chambre de lavage (2).

**8.** Procédé selon l'une quelconque des revendications précédentes, dans lequel la température à l'intérieur d'une chambre de lavage (2) du laveur désinfecteur (1) est de 65 degrés Celsius ou inférieure pendant le processus complet de stérilisation ou de désinfection dudit dispositif (D).

**9.** Procédé selon l'une quelconque des revendications précédentes, ledit procédé comprenant au moins les phases consécutives suivantes ;
au moins une phase de lavage pour traiter ledit dispositif (D) avec un détergeant,
au moins une phase de rinçage ultérieure au lavage pour rincer ledit dispositif (D) avec de l'eau,
au moins une phase de désinfection enzymatique pour traiter ledit dispositif (D) avec ladite haloperoxydase, ledit peroxyde d'hydrogène, lesdits ions chlorure et/ou bromure, et lesdits ions ammonium, et
au moins une phase de rinçage ultérieure à la désinfection enzymatique pour rincer ledit dispositif (D) avec de l'eau.

**10.** Laveur désinfecteur pour désinfecter ou stériliser un dispositif, **caractérisé en ce que** ledit laveur désinfecteur (1) comprend
une chambre de lavage (2) qui est adaptée pour abriter ledit dispositif (D), et
un agencement (34, 36, 38, 40, 42, 44) contenant une haloperoxydase, du peroxyde d'hydrogène, des ions chlorure et/ou bromure, et des ions ammonium, et agencé pour fournir ces substances à ladite chambre de lavage (2).

**11.** Laveur désinfecteur selon la revendication 10, ledit laveur désinfecteur (1) comprenant un agencement de fourniture d'haloperoxydase (34, 36, 38) qui fonctionne pour stocker et fournir une solution d'haloperoxydase contenant de l'haloperoxydase, et un agencement de fourniture de peroxyde d'hydrogène (40, 42, 44) qui fonctionne pour stocker et fournir une solution de peroxyde d'hydrogène contenant du peroxyde d'hydrogène et/ou un précurseur de peroxyde d'hydrogène.

**12.** Laveur désinfecteur selon l'une quelconque des revendications 10 - 11, ledit laveur désinfecteur (1) comprenant une unité de commande (62) qui fonctionne pour commander que la température à l'intérieur de ladite chambre de lavage (2) soit de 65 degrés Celsius ou inférieure pendant le processus complet de désinfection ou de stérilisation dudit dispositif (D).

**13.** Laveur désinfecteur selon l'une quelconque des revendications 10 - 12, ledit laveur désinfecteur (1) comprenant un agencement de fourniture d'eau (8, 10, 12), qui fonctionne pour fournir une eau propre, ayant passé au travers d'un filtre ayant une taille de pore de 0,2 micromètre au maximum, ou ayant au moins le degré correspondant de pureté, à la chambre de lavage (2) pour rincer ledit dispositif (D) après avoir été traité avec ladite haloperoxydase, ledit peroxyde d'hydrogène, lesdits ions chlorure et/ou bromure, et lesdits ions ammonium.

**14.** Laveur désinfecteur selon l'une quelconque des revendications 10 - 13, ledit laveur désinfecteur (1) comprenant un agencement de fourniture de détergeant (28, 30, 32) qui fonctionne pour fournir un détergeant à ladite chambre de lavage (2) avant de fournir ladite haloperoxydase, ledit peroxyde d'hydrogène, lesdits ions chlorure et/ou bromure, et lesdits ions ammonium à ladite chambre de lavage (2).

15. Utilisation d'une haloperoxydase, de peroxyde d'hydrogène, d'ions chlorure et/ou bromure, et d'ions ammonium dans un laveur désinfecteur (1) pour désinfecter ou stériliser un dispositif (D).

16. Utilisation selon la revendication 15, pour désinfecter un dispositif médical (D).

17. Utilisation selon l'une quelconque des revendications 15 et 16, dans laquelle la température à l'intérieur d'une chambre de lavage (2) du laveur désinfecteur (1) est de 65 degrés Celsius ou inférieure pendant la désinfection du dispositif (D).

EP 2 362 732 B1

Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007147536 A
- WO 2007147538 A
- WO 9638548 A
- WO 9527046 A
- US 5389369 A
- WO 0208377 A
- WO 9908531 A
- WO 9704102 A

- WO 0179459 A
- WO 0179458 A
- WO 0179461 A
- WO 0179460 A
- US 6248575 B
- WO 9529996 A
- WO 0050606 A
- WO 9931990 A

**Non-patent literature cited in the description**

- Class II Special Controls Guidance Document: Medical Washers and Medical Washer-Disinfectors; Guidance for the Medical Device Industry and FDA Review Staff. *U.S. Food and Drug Administration,* February 2002
- *U.S. Food and Drug Administration,* February 2002

- *Sterilized Water of the United States Pharmacopeia,* 01 January 1995
- Guidelines for Drinking Water Quality. WHO
- Rapid colorimetric assay for cellular growth and survival: Application to profileration and cytotoxicity assays. *J. Imm. Meth,* 1983, vol. 65, 55-63